# EUROPEAN PATENT APPLICATION

(11) **EP 4 462 098 A1**
(43) Date of publication of application: **13.11.2024**
(21) Application number: 22922201.3
(22) Date of filing: 23.12.2022
(51) Int. Cl.: G01N 1/28, C12M 1/00, C12M 1/26, C12M 3/00, C12N 11/02, G01N 1/00, G01N 1/04

(54) **GEL SHEET CHIP, METHOD FOR MANUFACTURING GEL SHEET CHIP, SAMPLE COLLECTING METHOD, GEL PLATE, SAMPLE COLLECTING SET, CULTURE IMPLEMENT, AND METHOD FOR MANUFACTURING CULTURE IMPLEMENT**

(30) Priority: 18.01.2022 JP 2022005679; 18.01.2022 JP 2022005680; 17.10.2022 JP 2022166452
(71) Applicant: NTN Corporation, Osaka-shi Osaka 530-0005 (JP)
(72) Inventor: TSUKAMOTO, Yoshinari, Iwata-shi, Shizuoka 438-8510 (JP); CHIKAE, Shohei, Iwata-shi, Shizuoka 438-8510 (JP)
(74) Representative: Bockhorni & Brüntjen Partnerschaft Patentanwälte mbB
(86) International application number: PCT/JP2022/047709
(87) International publication number: WO 2023/140051

(57) **Abstract**

A gel sheet chip (6) includes a film (2), a frame (3), gel (4D), and a sample (4B) to be collected. The frame (3) is placed on the film (2) and a through-hole (3A) is formed in the frame (3). The gel (4D) is disposed on the film (3) in the through-hole (3A). The sample (4B) to be collected is supported by the gel (4D).

## Description

### TECHNICAL FIELD

The present disclosure relates to a gel sheet chip, a method for manufacturing the gel sheet chip, a sample collecting method, a gel plate, a sample collecting set, a culture implement, and a method for manufacturing the culture implement.

### BACKGROUND ART

In recent years, a technique of selecting a target cell and a cell population with an analysis using a single cell has been rapidly growing. As the technique of selecting a target cell, cell selection methods such as a limiting dilution method, a cell sorter, a microfluidic channel, and a colony pick-up method have been attracting attention.

As a technique of handling a small amount of cells, a printing technique for handling a small amount of droplet has been attracting attention. As the printing technique, there are an inkjet scheme, a dispenser scheme, and an application scheme.

However, it is sometimes preferable to collect only one cell. From the viewpoint of appropriately collecting only any one cell, a technique of highly accurately extracting a further minute amount of droplets than the printing technique described above is required. From this viewpoint, a method using a manipulator disclosed in Japanese Patent Laying Open No. 2008-152044 (Patent Literature 1) and a method for cutting and collecting a biological specimen such as a cell using a collecting needle disclosed in WO2017/061387 (Patent Literature 2) have been developed.

### CITATION LIST

### PATENT LITERATURE

PTL 1: Japanese Patent Laying Open No. 2008-152044
PTL 2: WO2017/061387

### SUMMARY OF INVENTION

### TECHNICAL PROBLEM

In Japanese Patent Laying Open No. 2008-152044, a solution containing a cell to be collected is inhaled into a hollow portion of a tube. Since the cell to be collected is contained in the solution, when the solution is ejected into a container, the cell scatters on the inside of the container. For this reason, there is a problem in that sample collection with high stability and reproducibility is difficult.

In WO2017/061387, gel comes into contact with a cell specimen. The cell specimen cut is stored first in the collecting needle. Thereafter, liquid is injected into the collecting needle. The cell specimen in the collecting needle is discharged to the outside of the collecting needle by the pressure of the liquid. However, the gel is not fixedly supported to surround the periphery of the cell. For this reason, in particular, if the cell is a cell nonadherent to the gel, in a specimen of the cell, collapse and scattering of the sample occur at the time of the discharge to the outside of the collecting needle. It is also likely that the collecting needle pricks the cell to thereby damage the cell. The position of the cell is not fixed by the gel. It is difficult to collect the cell with high accuracy and good reproducibility.

The present disclosure has been made in view of the problems described above. An object of the present disclosure is to provide a gel sheet chip, a sample collecting set, and a method for manufacturing the gel sheet chip used in order to perform sample collection with high stability, reproducibility, and position accuracy.

An object of the present disclosure is to provide a sample collecting method and a gel plate and a sample collecting set used in the method capable of performing sample collection with high stability, reproducibility, and position accuracy.

An object of the present disclosure is to provide a method for manufacturing a highly accurate culture implement and the culture implement by making it possible to more easily control a concentration gradient of a factor.

### SOLUTION TO PROBLEM

A sample to be collected is fixed to a gel sheet chip to which the sample to be collected is fixed according to the present disclosure. The gel sheet chip includes a film, a frame, gel, and the sample. The frame is placed on the film and a through-hole is formed in the frame. The gel is disposed on the film in the through-hole. The sample to be collected is supported by the gel.

In a method for manufacturing a gel sheet chip to which a sample to be collected is fixed according to the present disclosure, a frame in which a through-hole is formed is installed on a film. A fluid sample including the sample to be collected is supplied into the through-hole on the film. The thickness of the fluid sample is uniformized by sandwiching a structure including the film, the frame, and the fluid sample with flat plates installed on the upper side and the lower side of the structure. The fluid sample is gelatinized.

A sample collecting set for fixing a sample to be collected according to the present disclosure includes a gel material, a film, and a frame. The gel material is capable of, by forming gel, supporting the sample to be collected. The gel can be placed on the film. The frame can be placed on the film. A through-hole is formed in the frame.

In a sample collecting method for collecting a fixed sample to be collected according to the present disclosure, gel that supports the sample to be collected is formed on a film in a sheet state and a gel sheet is provided on the film. The gel sheet on the film is punctured by a needle including a hollow shaped section, whereby a part of the gel sheet is cut and a cut portion of the gel sheet is stored in the hollow shaped section. The portion of the gel sheet is collected by discharging the portion of the gel sheet in the hollow shaped section from the inside of the hollow shaped section.

A gel plate to which a sample to be collected is fixed according to the present disclosure includes a film and a gel sheet. The gel sheet is formed on the film in a sheet state and includes gel that supports the sample to be collected.

A sample collecting set for fixing a sample to be collected according to the present disclosure includes a gel material and a film. The gel material is capable of, by forming gel, supporting the sample to be collected. The gel can be placed on the film.

In a method for manufacturing a culture implement according to the present disclosure, a gel sheet is provided by forming, on a film, in a sheet state, gel containing a factor to be supplied. The gel sheet on the film is punctured by a needle including a hollow shaped section to cut a part of the gel sheet. Solid gel, which is a cut portion of the gel sheet, is stored in the hollow shaped section. By discharging the solid gel in the hollow shaped section from the inside of the hollow shaped section, the solid gel is supplied into, as a sustained-release material including the factor, a container in which a cell is cultured.

In a culture implement according to the present disclosure, a cell is cultured. The culture implement includes a container, solid gel, and a medium. The solid gel is fixed in the container and a factor is contained in the solid gel. The medium fills at least a part in the container and the cell is cultured. The solid gel is disposed to be covered with the medium. The solid gel has a pillar shape.

### ADVANTAGEOUS EFFECTS OF INVENTION

According to the present disclosure, it is possible to provide a gel sheet chip, a sample collecting set, and a method for manufacturing the gel sheet chip capable of highly accurately performing sample collection with high stability, reproducibility, and position accuracy.

According to the present disclosure, it is possible to provide a sample collecting method, a gel plate, and a sample collecting set capable of performing sample collection with high stability, reproducibility, and position accuracy.

According to the present disclosure, it is possible to provide a method for manufacturing a culture implement and the culture implement by making it possible to more easily control a concentration gradient of a factor.

### BRIEF DESCRIPTION OF DRAWINGS

Fig. 1 is a schematic front view of an inside of a sample collecting apparatus according to a first embodiment.
Fig. 2 is a schematic diagram illustrating a collecting mechanism of the sample collecting apparatus illustrated in Fig. 1.
Fig. 3 is a schematic diagram for explaining a configuration of a collecting needle holder in the collecting mechanism illustrated in Fig. 2.
Fig. 4 is a schematic perspective view of a film and a frame forming a gel sheet chip according to the first embodiment.
Fig. 5 is a schematic perspective view illustrating a form in which the frame is placed on the film illustrated in Fig. 4.
Fig. 6 is a schematic sectional view of a portion extending along VI-VI line in Fig. 5.
Fig. 7 is a schematic perspective view of a gel sheet chip according to a first example of the first embodiment.
Fig. 8 is a schematic sectional view illustrating a first step of a method for manufacturing the gel sheet chip according to the first example of the first embodiment.
Fig. 9 is a schematic sectional view illustrating a second step of the method for manufacturing the gel sheet chip according to the first example of the first embodiment.
Fig. 10 is a schematic sectional view illustrating a third step of the method for manufacturing the gel sheet chip according to the first example of the first embodiment.
Fig. 11 is a schematic sectional view illustrating a fourth step of the method for manufacturing the gel sheet chip according to the first example of the first embodiment.
Fig. 12 is a schematic perspective view of a gel sheet chip according to a second example of the first embodiment.
Fig. 13 is a schematic sectional view illustrating a first step of a method for manufacturing the gel sheet chip according to the second example of the first embodiment.
Fig. 14 is a schematic sectional view illustrating a second step of the method for manufacturing the gel sheet chip according to the second example of the first embodiment.
Fig. 15 is a schematic sectional view illustrating a third step of the method for manufacturing the gel sheet chip according to the second example of the first embodiment.
Fig. 16 is a schematic sectional view illustrating a fourth step of the method for manufacturing the gel sheet chip according to the second example of the first embodiment.
Fig. 17 is a schematic perspective view of a gel sheet chip according to a third example of the first embodiment.
Fig. 18 is a schematic sectional view illustrating a first step of a supporting method in an example in which gel supports a sample with embedding.
Fig. 19 is a schematic sectional view illustrating a second step of the supporting method in the example in which the gel supports the sample with the embedding.
Fig. 20 is a schematic perspective view illustrating a first step of a supporting method in an example in which gel supports a sample with bonding.
Fig. 21 is a schematic perspective view illustrating a second step of the supporting method in the example in which the gel supports the sample with the bonding.
Fig. 22 is a schematic sectional view of a portion extending along XXII-XXII line in Fig. 21.
Fig. 23 is a schematic sectional view illustrating a third step of the supporting method in the example in which the gel supports the sample with the bonding.
Fig. 24 is a schematic sectional view illustrating a fourth step of the supporting method in the example in which the gel supports the sample with the bonding.
Fig. 25 is a schematic diagram illustrating a modification of Fig. 24.
Fig. 26 is a schematic diagram illustrating a sample collecting set for fixing a sample to be collected according to the first embodiment.
Fig. 27 is a schematic diagram illustrating a first step of a sample collecting method using a gel sheet chip in which gel supports a sample with embedding.
Fig. 28 is a schematic diagram illustrating a second step of the sample collecting method using the gel sheet chip in which the gel supports the sample with the embedding.
Fig. 29 is a schematic diagram illustrating a third step of the sample collecting method using the gel sheet chip in which the gel supports the sample with the embedding.
Fig. 30 is a schematic diagram illustrating a first step of a sample collecting method using a gel sheet chip in which gel supports a sample with bonding.
Fig. 31 is a schematic diagram illustrating a second step of the sample collecting method using the gel sheet chip in which the gel supports the sample with the bonding.
Fig. 32 is a schematic diagram illustrating a third step of the sample collecting method using the gel sheet chip in which the gel supports the sample with the bonding.
Fig. 33 is a schematic sectional view illustrating a first example of a step of recovering only a cell in which gel is dissolved from a gel sheet portion.
Fig. 34 is a schematic sectional view illustrating a second example of the step of recovering only the cell in which the gel is dissolved from the gel sheet portion.
Fig. 35 is a schematic front view of the inside of a sample collecting apparatus according to a second embodiment.
Fig. 36 is a schematic sectional view illustrating a first step of a sample collecting method according to the second embodiment.
Fig. 37 is a schematic sectional view illustrating a second step of the sample collecting method according to the second embodiment.
Fig. 38 is a schematic sectional view illustrating a third step of the sample collecting method according to the second embodiment.
Fig. 39 is a schematic sectional view illustrating a fourth step of the sample collecting method according to the second embodiment.
Fig. 40 is a schematic perspective view of a gel plate.
Fig. 41 is a schematic sectional view illustrating a fifth step of the sample collecting method according to the second embodiment.
Fig. 42 is a schematic enlarged perspective view illustrating a plate and a gel plate on the plate included in Fig. 41.
Fig. 43 is a schematic sectional view illustrating a sixth step of the sample collecting method according to the second embodiment.
Fig. 44 is a schematic enlarged sectional view of a region XLIV surrounded by a dotted line in Fig. 43.
Fig. 45 is a schematic sectional view illustrating a seventh step of the sample collecting method according to the second embodiment.
Fig. 46 is a schematic enlarged sectional view of a region XLVI surrounded by a dotted line in Fig. 45.
Fig. 47 is a schematic sectional view illustrating an eighth step of the sample collecting method according to the second embodiment.
Fig. 48 is a schematic sectional view illustrating a first modification of the second embodiment.
Fig. 49 is a schematic sectional view illustrating a second modification of the second embodiment.
Fig. 50 is a schematic sectional view illustrating a third modification of the second embodiment.
Fig. 51 is a schematic sectional view illustrating a step in which gel obtained from a fluid sample illustrated in Fig. 50 is cut.
Fig. 52 is a schematic diagram illustrating a sample collecting set for fixing a sample to be collected according to the second embodiment.
Fig. 53 is a schematic sectional view illustrating an example of a step of recovering only a cell in which gel is dissolved from a gel sheet portion.
Fig. 54 is a schematic sectional view illustrating a first step of a sample collecting method according to a third embodiment.
Fig. 55 is a schematic sectional view illustrating a second step of the sample collecting method according to the third embodiment.
Fig. 56 is a schematic sectional view illustrating a third step of the sample collecting method according to the third embodiment.
Fig. 57 is a schematic sectional view illustrating a fourth step of the sample collecting method according to the third embodiment.
Fig. 58 is a schematic perspective view of a gel plate.
Fig. 59 is a schematic sectional view illustrating a fifth step of the sample collecting method according to the third embodiment.
Fig. 60 is a schematic enlarged perspective view illustrating a plate and a gel plate on the plate included in Fig. 59.
Fig. 61 is a schematic sectional view illustrating a sixth step of the sample collecting method according to the third embodiment.
Fig. 62 is a schematic enlarged sectional view of a region LXII surrounded by a dotted line in Fig. 61.
Fig. 63 is a schematic sectional view illustrating a seventh step of the sample collecting method according to the third embodiment.
Fig. 64 is a schematic enlarged sectional view of a region LXIV surrounded by a dotted line in Fig. 63.
Fig. 65 is a schematic sectional view illustrating an eighth step of the sample collecting method according to the third embodiment.
Fig. 66 is a schematic sectional view illustrating a modification of the eighth step illustrated in Fig. 65.
Fig. 67 is a schematic sectional view illustrating a step equivalent to the eighth step illustrated in Fig. 65 in a first example of supply of solid gel into a container.
Fig. 68 is a schematic sectional view illustrating a culture implement formed by a step following Fig. 67 in the first example of the supply of the solid gel into the container.
Fig. 69 is a schematic sectional view illustrating a step equivalent to a pre-stage of the eighth step illustrated in Fig. 65 in a second example of the supply of the solid gel into the container.
Fig. 70 is a schematic sectional view illustrating a step equivalent to the eighth step illustrated in Fig. 65 in the second example of the supply of the solid gel into the container.
Fig. 71 is a schematic sectional view illustrating a step following Fig. 70 in the second example of the supply of the solid gel into the container.
Fig. 72 is a schematic sectional view illustrating a culture implement formed by a step following Fig. 71 in the second example of the supply of the solid gel into the container.
Fig. 73 is a schematic sectional view illustrating a step equivalent to the pre-stage of the eighth step illustrated in Fig. 65 in a third example of the supply of the solid gel into the container.
Fig. 74 is a schematic sectional view illustrating a step equivalent to the eighth step illustrated in Fig. 65 in the third example of the supply of the solid gel into the container.
Fig. 75 is a schematic sectional view illustrating a step following Fig. 74 in the third example of the supply of the solid gel into the container.
Fig. 76 is a schematic sectional view illustrating a culture implement formed by a step following Fig. 75 in the third example of the supply of the solid gel into the container.
Fig. 77 is a schematic sectional view illustrating a step equivalent to Fig. 74 in a fourth example of the supply of the solid gel into the container.
Fig. 78 is a schematic sectional view illustrating a culture implement formed by a step equivalent to Fig. 76 in the fourth example of the supply of the solid gel into the container.
Fig. 79 is a schematic sectional view illustrating a step equivalent to a pre-stage of Fig. 77 in a fifth example of the supply of the solid gel into the container.
Fig. 80 is a schematic sectional view illustrating a step equivalent to Fig. 77 in the fifth example of the supply of the solid gel into the container.
Fig. 81 is a schematic sectional view illustrating a culture implement formed by a step equivalent to Fig. 78 in the fifth example of the supply of the solid gel into the container.

### DESCRIPTION OF EMBODIMENTS

### (First Embodiment)

Embodiments are explained below with reference to the drawings.

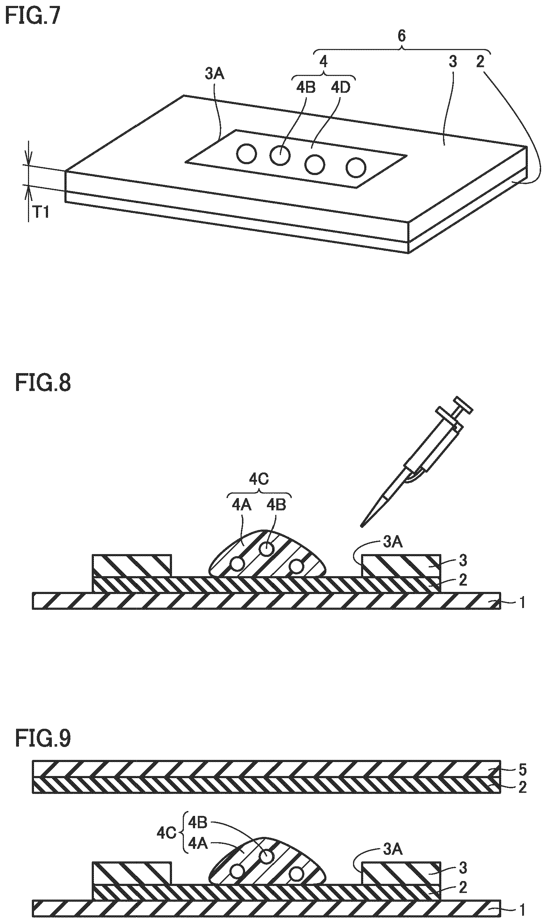

First, characteristics of the present embodiment are briefly explained. As illustrated in Fig. 7, a gel sheet chip 6 includes a film 2, a frame 3, gel 4D, and a sample 4B to be collected. Frame 3 is placed on film 2 and a through-hole 3A is formed in frame 3. Gel 4D is disposed on film 2 to be in contact with wall surfaces of through-hole 3A. Sample 4B is supported by gel 4D. The embodiment is explained in detail below.

### (Overall configuration of a sample collecting apparatus)

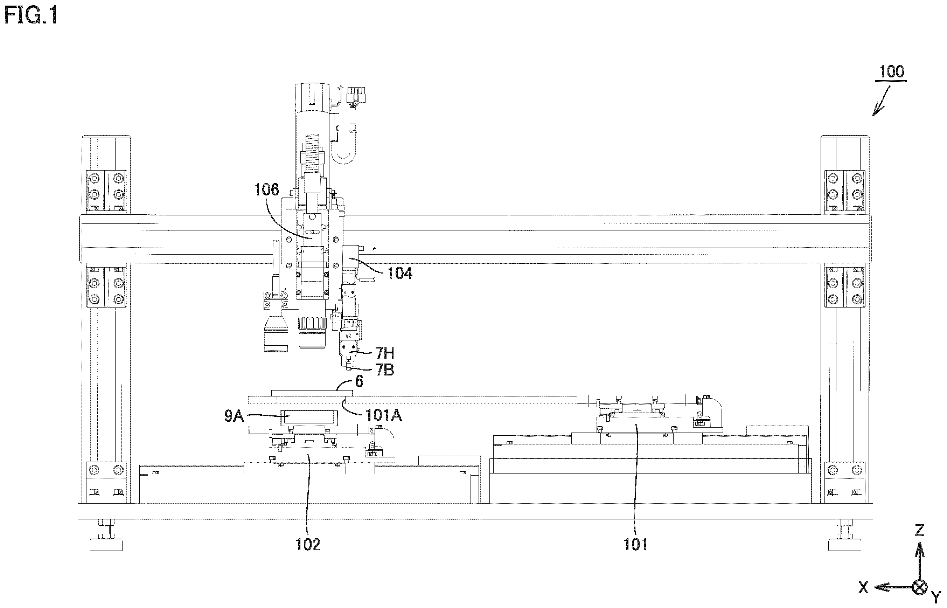

Fig. 1 is a schematic front view of the inside of a sample collecting apparatus according to a first embodiment. Note that, for convenience of explanation, an X direction, a Y direction, and a Z direction are introduced. Referring to Fig. 1, a sample collecting apparatus 100 is an apparatus for collecting a sample from gel sheet chip 6 serving as a sample explained below. Sample collecting apparatus 100 mainly includes a treatment chamber, an XY stage for sample 101 disposed on the inside of the treatment chamber, an XY stage for container 102, and a collecting mechanism 104. Note that sample collecting apparatus 100 illustrated in Fig. 1 includes only one collecting mechanism 104 but may include a plurality of collecting mechanisms 104.

XY stage for sample 101 (a stage for sample) is movable in the horizontal direction, that is, an XY direction (the X direction and the Y direction). Specifically, for example, a guide section is installed on the lower surface of XY stage for sample 101. The guide section is slidably connected to a guide rail installed on the bottom surface of the treatment chamber. An upper part surface of XY stage for sample 101 is formed as a mounting surface on which gel sheet chip 6 can be fixed. A stage penetrating section 101A penetrating XY stage for sample 101 is formed in at least a part of XY stage for sample 101. Gel sheet chip 6 is preferably fixed to a position overlapping stage penetrating section 101A.

XY stage for container 102 (a stage for container) is disposed below XY stage for sample 101. XY stage for container 102 is movable in the horizontal direction, that is, the XY direction (the X direction and the Y direction). Specifically, for example, a guide section is installed on the lower surface of XY stage for container 102. The guide section is slidably connected to the guide rail installed on the bottom surface of the treatment chamber. XY stage for container 102 fixes a container 9A in which a part of gel sheet chip 6 can be stored. Accordingly, in Fig. 1, collecting mechanism 104, gel sheet chip 6, and container 9A are disposed in this order from the upper side to the lower side of the Z direction.

Collecting mechanism 104 and an observation optical system 106 are connected to, for example, a Z-axis table that is a member movable in the Z direction. That is, collecting mechanism 104 and observation optical system 106 are held to be movable in the Z direction in sample collecting apparatus 100. Observation optical system 106 observes and measures a position and the like of a sample to be collected included in gel sheet chip 6. In observation optical system 106, a CCD camera that converts an observed image into an electric signal may be installed. The observation of gel sheet chip 6 and the like by observation optical system 106 may be performed by visible light. However, the observation of gel sheet chip 6 and the like may be performed by not only the visible light but also an infrared ray, an X ray, ultrasound, or the like. The observation of gel sheet chip 6 is possible using magnetism depending on the material of gel sheet chip 6. Gel sheet chip 6 observed by means other than the visible light does not need to be transparent or semitransparent and may be nontransparent.

Although not illustrated, a control unit is installed on the outside of the treatment chamber. The control unit includes a monitor, a computer for control, and an operation panel. In the control unit, a command value of operation speed of a needle for collection from the operation panel is input to and stored in a storage device of the computer for control. For example, at a sample collecting operation time, a command value of speed of the sample collecting operation is read from the storage device and sent to a control program of collecting mechanism 104. A control program of collecting mechanism 104 determines, based on the command value, rotating speed of a servomotor 41 explained below included in collecting mechanism 104 and moves a collecting needle unit 24 up and down at predetermined speed. Accordingly, a collecting operation and the like of collecting mechanism 104 are performed. When the computer for control is communicating with a control system further on an upstream side, the command value may be received from the control system. Parameters corresponding to the material of a gel sheet to be collected as a sample may be stored in the storage device. A command value of speed of a collecting operation may be calculated according to a designated material, a designated thickness, and a designated collecting method for the gel sheet.

### (Configurations of the collecting mechanism and a collecting needle holder)

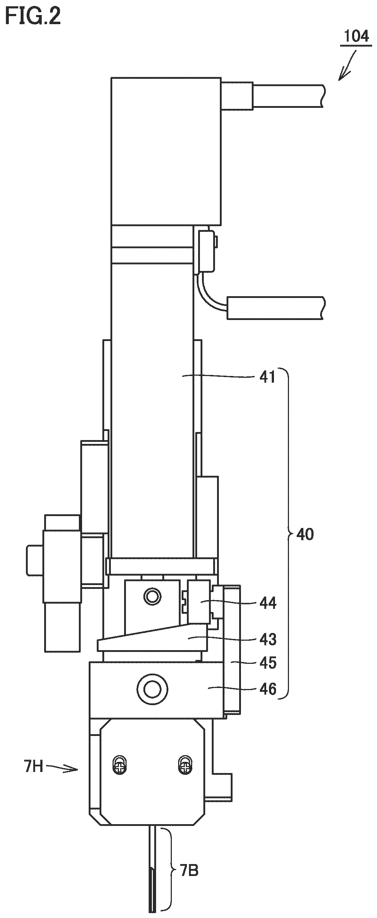

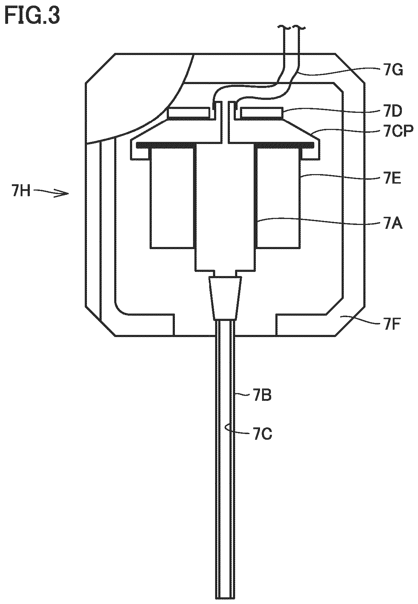

Fig. 2 is a schematic diagram illustrating the collecting mechanism of the sample collecting apparatus illustrated in Fig. 1. Fig. 3 is a schematic diagram for explaining a configuration of a collecting needle holder in the collecting mechanism illustrated in Fig. 2. Collecting mechanism 104 explained above is explained more in detail with reference to Fig. 2 and Fig. 3.

Collecting mechanism 104 illustrated in Fig. 1 includes a collecting needle holder 7H including a pin 7B that is a collecting needle. Collecting mechanism 104 includes a first driving unit 40. Collecting needle holder 7H is detachably connected to first driving unit 40. As a structure for connecting collecting needle holder 7H to first driving unit 40, any structure can be adopted.

Any configuration can be adopted for first driving unit 40. For example, as illustrated in Fig. 2, first driving unit 40 includes a servomotor 41, a cam 43, a bearing 44, a cam coupling plate 45, and a movable unit 46. Servomotor 41 is installed such that, for example, a rotating shaft extends in a direction along the Z direction illustrated in Fig. 1. Cam 43 is connected to the rotating shaft of servomotor 41. Cam 43 is capable of rotating centering on the rotating shaft of servomotor 41.

Cam 43 includes a center section connected to the rotating shaft of servomotor 41 and a flange section connected to one end portion of the center section. An upper part surface (a surface on servomotor 41 side) of the flange section is formed as a cam surface. The cam surface is formed in a ring shape along the outer circumference of the center section. As illustrated in Fig. 2, the cam surface is formed in a slope shape such that the distance from the bottom surface of the flange section fluctuates. For example, the cam surface includes an upper end flat region having the largest distance from the bottom surface of the flange section, a lower end flat region disposed at an interval from the upper end flat region and having the smallest distance from the bottom surface of the flange section, and a slope section that connects the upper end flat region and the lower end flat region.

Bearing 44 is disposed to be in contact with the cam surface of cam 43. Bearing 44 is disposed in a specific direction (on the right side of servomotor 41) when viewed from cam 43 as illustrated in Fig. 2. When cam 43 rotates as the rotating shaft of servomotor 41 rotates, bearing 44 keeps a state in which bearing 44 is in contact with the cam surface. Cam coupling plate 45 is connected to bearing 44. In cam coupling plate 45, the other end portion on the opposite side to one end portion connected to bearing 44 is fixed to movable unit 46. A collecting needle holder storing unit is connected to movable unit 46. Collecting needle holder 7H explained above is stored in the collecting needle holder storing unit. For example, a first connecting member such as a magnet may be disposed on a surface facing the collecting needle holder storing unit in collecting needle holder 7H. A second connecting member such as a magnet or a magnetic body that can fix the first connecting member on collecting needle holder 7H side explained above may be disposed on the collecting needle holder storing unit side as well.

A fixing pin is installed in movable unit 46. The other fixing pin is installed in a stand that holds servomotor 41. A spring is installed to connect the two fixing pins. By the spring, movable unit 46 is in a state in which movable unit 46 receives a tensile force to the lower side. The tensile force by the spring acts on bearing 44 via movable unit 46 and cam coupling plate 45. With the tensile force of the spring, bearing 44 maintains a state in which bearing 44 is pressed against the cam surface of cam 43.

Movable unit 46 and the collecting needle holder storing unit are connected to a linear guide installed on the stand. The linear guide is disposed to extend in the Z direction. Therefore, movable unit 46 and the collecting needle holder storing unit (including pin 7B and collecting needle holder 7H) is movable in the Z direction.

Fig. 3 is a schematic diagram for explaining a configuration of the collecting needle holder in the collecting mechanism illustrated in Fig. 2. Referring to Fig. 3, collecting needle holder 7H is capable of griping an implement including a syringe 7A and pin 7B and playing a central role of performing collection. Pin 7B includes a hollow, that is, a hollow shaped section 7C on the inside thereof. Pin 7B falls to be able to cut and penetrate a target object.

Pin 7B is fixed to syringe 7A. In collecting needle holder 7H, syringe 7A is fixed by an upper syringe fixing section 7D and a lower syringe fixing section 7E. A syringe cap 7CP is attached to the upper side of syringe 7A. Tube 7G is connected to the upper side of syringe cap 7CP. Gas or liquid is fed into syringe 7A from tube 7G via syringe cap 7CP. Accordingly, a cut part of gel sheet chip 6 stored in hollow shaped section 7C of pin 7B is pressed downward and discharged to the outside of pin 7B.

Collecting needle holder 7H stores the members explained above with a holder outer shell 7F. However, parts of pin 7B and tube 7G may project to the outer side of holder outer shell 7F.

### (Gel sheet chip and a method for manufacturing the gel sheet chip)

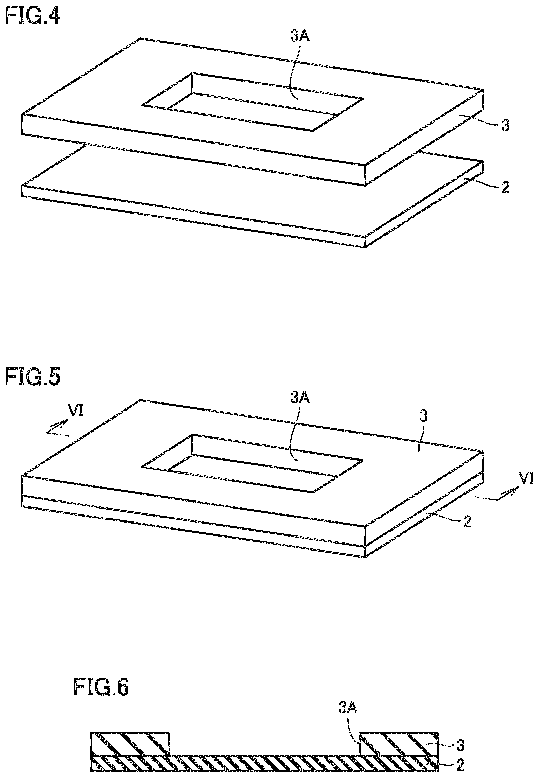

Fig. 4 is a schematic perspective view of the film and the frame forming the gel sheet chip according to the first embodiment. Fig. 5 is a schematic perspective view illustrating a form in which the frame is placed on the film illustrated in Fig. 4. Fig. 6 is a schematic sectional view of a portion extending along VI-VI line in Fig. 5. Referring to Fig. 4 to Fig. 6, the gel sheet chip according to the present embodiment includes film 2 and frame 3.

Film 2 is, for example, a thin film having a rectangular shape in plan view. Film 2 may be formed by any material selected out of a group consisting of polyethylene, polyvinylidene chloride, and polyvinyl chloride. Alternatively, film 2 may be formed by polydimethylsiloxane or butyl rubber. The thickness of film 2 is preferably 1 mm or less. In particular, the thickness of film 2 is preferably 10 µm or more and 100 µm or less. From the viewpoint explained above, a maximum elongation rate (an extreme elongation rate) of film 2 is preferably 100% or more and 1300% or less. By forming film 2 from the material and in the size explained above, film 2 has a characteristic that film 2 is soft and fragile enough to be easily damaged by small pressing due to falling of a pin explained below but a portion other than a punctured portion by the pin is not greatly torn and capable of supporting the gel sheet and the like even after the penetration. That is, the punctured portion of film 2 is more easily damaged by the falling of pin 7B to prevent damage to the portion other than the punctured portion. Further, in order to visually recognize the position of sample 4B through film 2, film 2 is preferably transparent or semitransparent suitable for observation by an optical system.

For example, frame 3 is preferably has substantially the same shape and the same size as the shape and the size of film 2 in plan view. That is, like film 2, frame 3 has a rectangular shape as an outer shape. As illustrated in Fig. 5 and Fig. 6, frame 3 is placed on film 2. A partial region (in plan view), that is, the center in plan view of frame 3 is penetrated. That is, through-hole 3A is formed in frame 3. Through-hole 3A is hollow inside and a material forming frame 3 is not disposed in through-hole 3A. Like the outer shape of frame 3, through-hole 3A may have a rectangular shape. Through-hole 3A and the outer shape of frame 3 may have similar shapes each other. The positions of the center of through-hole 3A and the center of entire frame 3 may overlap. The size of through-hole 3A is determined according to the size of a gel sheet 4 (see Fig. 7) determined according to a type and an amount of sample 4B to be embedded.

Frame 3 is preferably a material having high rigidity and capable of supporting film 2 and gel sheet 4 (see Fig. 7) from below. Specifically, frame 3 may be formed by any one selected out of a group consisting of a plastic material such as polystyrene, glass, metal, a silicon rubber-based material, and a nonwoven material.

Fig. 7 is a schematic perspective view of a gel sheet chip according to a first example of the first embodiment. Referring to Fig. 7, gel sheet chip 6 includes a gel sheet 4 besides film 2 and frame 3 explained above. Gel sheet 4 includes sample 4B and gel 4D. Sample 4B is a cell or the like to be collected. In Fig. 7, cells as a plurality of samples 4B are disposed one by one at intervals from one another.
Sample 4B is supported by solidified gel 4D. Gel 4D is solidified but is soft enough to be easily damaged by puncture by a pin. Note that sample 4B being supported by gel 4D means, for example, a state in which the periphery of sample 4B is embedded to be covered and buried by hard gel 4D and sample 4B does not flow. Therefore, in Fig. 7, sample 4B is disposed to be stored on the inside of gel 4D.

In gel sheet chip 6 illustrated in Fig. 7, the thickness of gel 4D is substantially the same as thickness T1 of frame 3. Here, substantially the same thickness includes not only a case in which the thicknesses are completely the same dimension but also a case in which the difference between an average value of the thickness of gel 4D and an average value of the thickness of frame 3 is 10% or less of the average value of the thickness of frame 3.

Gel 4D supporting sample 4B with, for example, embedding is disposed on film 2 in through-hole 3A of frame 3. Gel 4D is preferably disposed to be in contact with four wall surfaces (side surfaces as inner wall surfaces) of through-hole 3A. At least parts (only parts) of the four wall surfaces of through-hole 3A may be in contact with a wall surface of gel 4D. The entire four wall surfaces of through-hole 3A may be in contact with the wall surface of gel 4D. Frame 3 is placed on film 2, whereby the lowest part of through-hole 3A is covered with film 2. A container-like region is formed by film 2 covering through-hole 3A and the wall surfaces of through-hole 3A. Gel 4D is stored in the container-like region to be in contact with both of the wall surfaces of through-hole 3A and film 2. Accordingly, gel sheet chip 6 is formed.

Gel 4D is gel obtained by gelatinizing a gel material having biocompatibility. Here, having biocompatibility means that the gel material does not show toxicity such as inflammation even if a biological tissue such as a cell comes into contact with the gel material and has a nature with low invasiveness to the cell. Accordingly, it is possible to suppress damage to the cell (sample 4B) due to gel. It can be confirmed that a certain material has biocompatibility by the fact that the number of living cells does not decrease when cells are cultured on the certain material, living cells and dead cells are dyed, and inflammatory markers expressed by the cells or contents eluted from the cells are measured. Alternatively, it can be confirmed that the certain material has biocompatibility by the fact that the expression of the inflammatory markers has not increased or the contents have not eluted from the cells. A gel material 4A is preferably transparent or semitransparent from the viewpoint of enabling observation of the position of embedded sample 4B. Further, gel 4D is preferably made of a material having adhesion to a cell. Having adhesion means that, after the cell is seeded on the surface of gel 4D and left to stand for four hours or more, the cell adheres to the surface of gel 4D.

From the viewpoint of satisfying the above, gel material 4A is any one selected out of a group consisting of collagen, gelatin, fibrin, sodium alginate, gellan gum, agarose, hyaluronic acid, chitin, chitosan, polyethylene glycol, polyvinyl alcohol, and 2-methacryloyloxyethyl phosphorylcholine (MPC polymer).

In gel sheet chip 6, film 2 and frame 3 are preferably pasted by an adhesive such as cyanoacrylate. This is because cyanoacrylate has low invasiveness to the cell that is sample 4B.

Next, a method for manufacturing gel sheet chip 6 illustrated in Fig. 7 is explained with reference to Fig. 8 to Fig. 11. Fig. 8 is a schematic sectional view illustrating a first step of a method for manufacturing the gel sheet chip according to the first example of the first embodiment. Referring to Fig. 8, film 2 is placed on a flat plate such as a slide glass 1. Frame 3 in which through-hole 3A is formed is pasted on film 2 by, for example, the adhesive explained above.

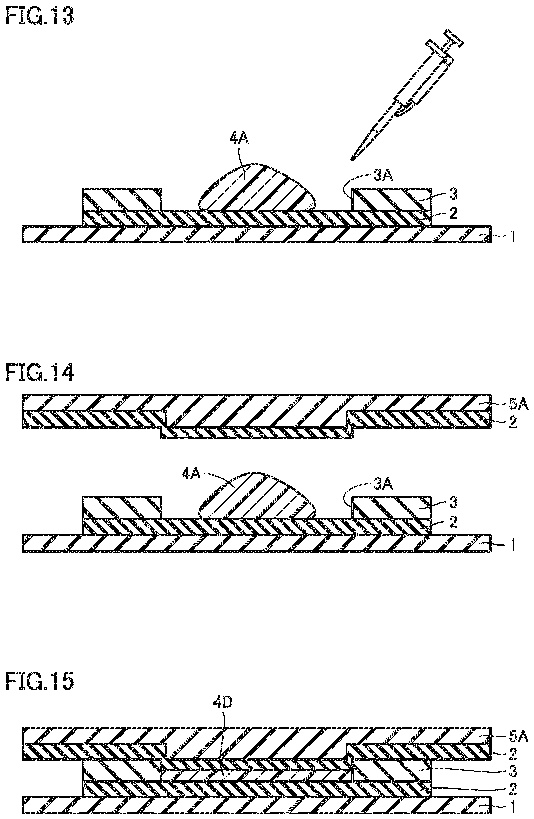

Next, a fluid sample 4C having fluidity in which one or more samples 4B are included in gel material 4A having fluidity capable of becoming gel is supplied into through-hole 3A on film 2. In Fig. 13, plurality of samples 4B are disposed one by one at intervals from one another.

Fig. 9 is a schematic sectional view illustrating a second step of the method for manufacturing the gel sheet chip according to the first example of the first embodiment. Referring to Fig. 9, film 2 is pasted to the lower surface of a slide glass 5. Slide glass 5 may be of the same material and size as slide glass 1 illustrated in Fig. 8. That is, slide glass 5 illustrated in Fig. 9 is a flat plate, the entire thickness of which is substantially uniform. Film 2 on the upper side of fluid sample 4C illustrated in Fig. 5 may be of the same material and size as film 2 illustrated in Fig. 4.

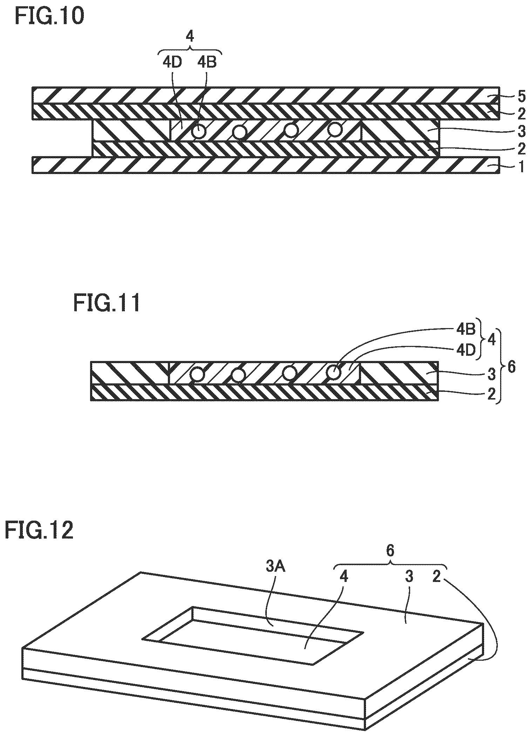

Fig. 10 is a schematic sectional view illustrating a third step of the method for manufacturing the gel sheet chip according to the first example of the first embodiment. Referring to Fig. 10, slide glass 5 to which film 2 is pasted is placed on frame 3. Film 2 pasted to slide glass 5 is in contact with fluid sample 4C and frame 3. Slide glass 5 and film 2 press fluid sample 4C and frame 3 downward. For this reason, a structure including film 2, frame 3, and fluid sample 4C (pasted to slide glass 1) on the lower side in Fig. 10 is sandwiched by slide glass 1 to which film 2 present under the structure is pasted and slide glass 5 to which film 2 present on the structure is pasted. Accordingly, slide glass 1, film 2, frame 3, and fluid sample 4C, film 2, and slide glass 5 are stacked in this order from the lower side to the upper side. The thickness of fluid sample 4C is uniformized by contact of a flat plate including slide glass 5 and film 2 from above. Here, the thickness being uniform is not limited to a case in which the thickness of fluid sample 4C is completely uniform in entire fluid sample 4C and means that the difference between a maximum value and a minimum value of the thickness of fluid sample 4C is 1% or less of an average value of the thickness of fluid sample 4C.

Next, for example, when gel material 4A is a material solidified depending on temperature while being left to stand in the state explained above, the entire stacked components explained above is cooled to, for example, 4°C. Gel material 4A forming fluid sample 4C is changed to solidified gel 4D by the cooling. Gel sheet 4 in which sample 4B is embedded on the inside of gel 4D is formed in through-hole 3A. As explained above, when gel material 4A is a material gelatinized by temperature, the structure explained above is left to stand under an optimum temperature environment. Although gel 4D is solidified, gel 4D is soft enough to be easily damaged by puncture by a pin.

Fig. 11 is a schematic sectional view illustrating a fourth step of the method for manufacturing the gel sheet chip according to the first example of the first embodiment. Referring to Fig. 11, after gel sheet 4 is formed, film 2 and slide glass 5 placed on the upper side of gel sheet 4 are removed and slide glass 1 is also removed. Accordingly, gel sheet chip 6 including film 2, frame 3, and gel sheet 4 placed on film 2 is formed. In other words, in Fig. 11, a sectional view of a form of gel sheet chip 6 illustrated in Fig. 7 is illustrated.

Fig. 12 is a schematic perspective view of a gel sheet chip according to a second example of the first embodiment. Referring to Fig. 12, gel sheet chip 6 has generally the same configuration as gel sheet chip 6 in the first example illustrated in Fig. 7. Therefore, the same members as the members illustrated in Fig. 7 are denoted by the same reference numerals and signs and explanation of the members is not repeated as long as functions of the members are the same. However, in gel sheet chip 6 illustrated in Fig. 12, gel 4D is thinner than frame 3. In particular, here, the difference between an average value of the thickness of gel 4D and an average value of the thickness of frame 3 exceeds 10% of an average value of the thickness of frame 3. For example, the average value of the thickness of gel 4D is a half of the average value of the thickness of frame 3. Gel 4D (including gel material 4A) illustrated in Fig. 12 (including Fig. 13 to Fig. 16) includes, as explained below, both of a case in which sample 4B is supported on the inside of gel 4D and a case in which sample 4B is supported to be bonded to the outside of gel 4D. From the viewpoint of including both of the cases, sample 4B is not illustrated in gel sheet 4 illustrated in Fig. 12. If the example illustrated in Fig. 7 and the example illustrated in Fig. 12 are put together, in the present embodiment, the thickness of gel 4D is equal to or less than the thickness of frame 3.

Next, a method for manufacturing gel sheet chip 6 illustrated in Fig. 12 is explained with reference to Fig. 13 to Fig. 16. However, explanation of portions overlapping the steps illustrated in Fig. 8 to Fig. 11 is not repeated. Fig. 13 is a schematic sectional view illustrating a first step of a method for manufacturing the gel sheet chip according to the second example of the first embodiment. Referring to Fig. 13, the same treatment as the treatment illustrated in Fig. 8 is basically performed. Gel material 4A having fluidity is supplied into through-hole 3A.

Fig. 14 is a schematic sectional view illustrating a second step of the method for manufacturing the gel sheet chip according to the second example of the first embodiment. Referring to Fig. 14, the same treatment as the treatment illustrated in Fig. 9 is basically performed. However, in a slide glass 5A illustrated in Fig. 14, the thickness of a region in through-hole 3A is larger compared with the thickness of a region other than the region in through-hole 3A. Specifically, the difference between the thickness of the region in through-hole 3A and the thickness of the other region of slide glass 5A is set to be equal to the difference between the thickness of frame 3 and the thickness of gel material 4A to be formed after compression.

Fig. 15 is a schematic sectional view illustrating a third step of the method for manufacturing the gel sheet chip according to the second example of the first embodiment. Referring to Fig. 15, the same treatment as the treatment illustrated in Fig. 10 is basically performed. However, in Fig. 15, slide glass 5A to which film 2 is pasted is placed on frame 3. Slide glass 5A and film 2 press gel material 4A and frame 3 downward. Slide glass 5A projects further downward in a region overlapping through-hole 3A than the other region. For this reason, the projecting portion presses gel material 4A while entering through-hole 3A. Accordingly, gel material 4A is thinner than frame 3.

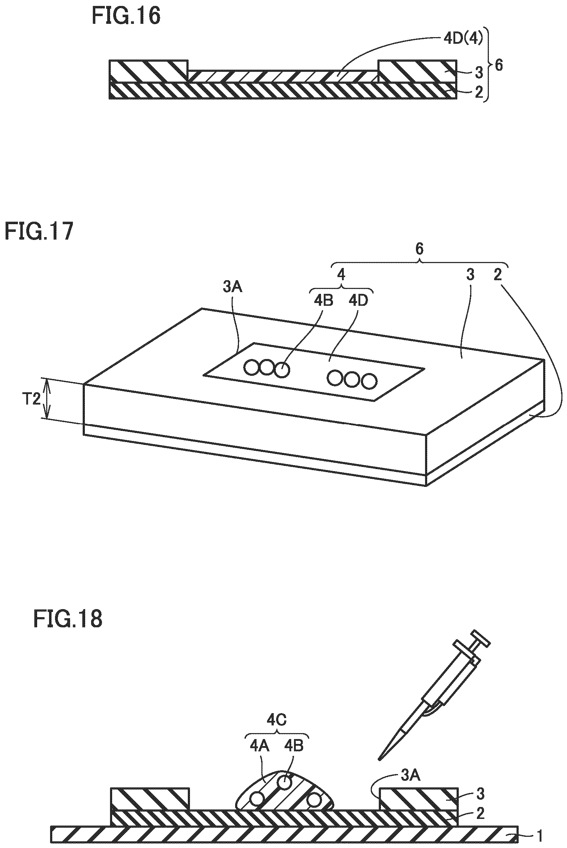

Fig. 16 is a schematic sectional view illustrating a fourth step of the method for manufacturing the gel sheet chip according to the second example of the first embodiment. Referring to Fig. 16, the same treatment as the treatment illustrated in Fig. 11 is basically performed. In Fig. 16, a sectional view of a form of gel sheet chip 6 illustrated in Fig. 11 is illustrated.

Fig. 17 is a schematic perspective view of a gel sheet chip according to a third example of the first embodiment. Referring to Fig. 17, gel sheet chip 6 has generally the same configuration as gel sheet chip 6 in the first example illustrated in Fig. 7. Therefore, the same members as the members illustrated in Fig. 7 are denoted by the same reference numerals and signs and explanation of the members is not repeated as long as functions of the members are the same. However, in gel sheet chip 6 illustrated in Fig. 17, the thickness of frame 3 is T2 and sample 4B is a cell aggregate. That is, plurality of samples 4B are gathered to be a mass. In this regard, Fig. 17 is different in a configuration from Fig. 7 in which samples 4B are separated one by one from one another and supported by gel 4D. The aggregate needs to have size capable of passing through a hollow shaped section of a pin explained below. That is, the aggregate is required to have size smaller than the width in a direction intersecting a direction in which the hollow shaped section extends. In Fig. 17, as an example, three samples 4B are gathered in the aggregate. However, not only this, but two or four or more samples 4B form the aggregate.

Next, a method for supporting sample 4B with gel 4D is explained with reference to Fig. 18 to Fig. 25.

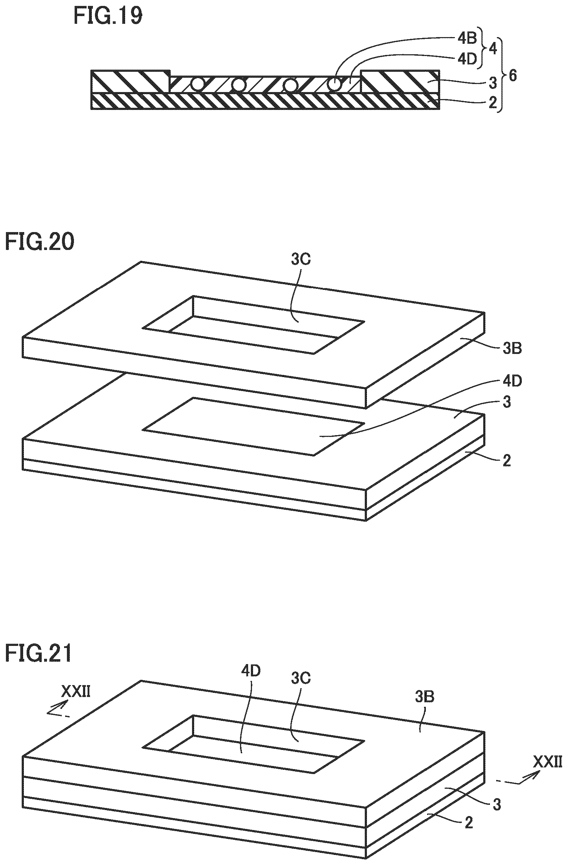

Fig. 18 is a schematic sectional view illustrating a first step of a supporting method in an example in which gel supports a sample with embedding. Fig. 19 is a schematic sectional view illustrating a second step of the supporting method in the example in which the gel supports the sample with the embedding. Referring to Fig. 18, when gel sheet chip 6 in which sample 4B is supported to be embedded in gel 4D is formed as illustrated in Fig. 7 to Fig. 11 (Fig. 12 to Fig. 16), as in Fig. 8, sample 4B is embedded in gel material 4A. Fig. 18 illustrates the same treatment as the treatment illustrated in Fig. 8. However, an amount of fluid sample 4C supplied into through-hole 3A on film 2 is smaller than the amount illustrated in Fig. 8. In this case, referring to Fig. 19, gel sheet chip 6 to be formed is generally the same as gel sheet chip 6 illustrated in Fig. 11. However, gel sheet 4 is formed thinner than gel sheet 4 illustrated in Fig. 11. That is, in Fig. 19, gel sheet 4 is formed thinner than frame 3 illustrated in Fig. 12 and Fig. 16. Fig. 19 is an example in which gel sheet 4 is disposed such that sample 4B is embedded in gel 4D.

In the example in which sample 4B is embedded in gel 4D, the first example illustrated in Fig. 7 to Fig. 11 (gel sheet 4 has substantially the same thickness as frame 3) may be applied or the example illustrated in Fig. 18 and Fig. 19 (gel sheet 4 is thinner than frame 3) may be applied.

In the example in which sample 4B is supported to be embedded in gel 4D as illustrated in Fig. 18 to Fig. 19 (including Fig. 7 to Fig. 11), the cell, which is sample 4B, may be non-adhesive or may be adhesive to gel 4D. Conversely speaking, gel 4D may be made of a material having adhesion to the cell or may not be such a material.

Fig. 20 is a schematic perspective view illustrating a first step of a supporting method in an example in which gel supports a sample with bonding. Referring to Fig. 20, a gel sheet chip including a gel sheet including only gel 4D not including sample 4B is formed by, for example, the same method as the method illustrated in Fig. 8 to Fig. 11 (except that sample 4B is included in gel material 4A). Note that, in Fig. 20, the thickness of gel 4D is substantially equal to the thickness of frame 3. However, in Fig. 20, gel 4D thinner than frame 3 may be formed by the same method as the method illustrated in Fig. 12 to Fig. 16. Note that, in Fig. 20, gel 4D is preferably made of a material having adhesion to a cell. Gel 4D is formed by, for example, collagen.

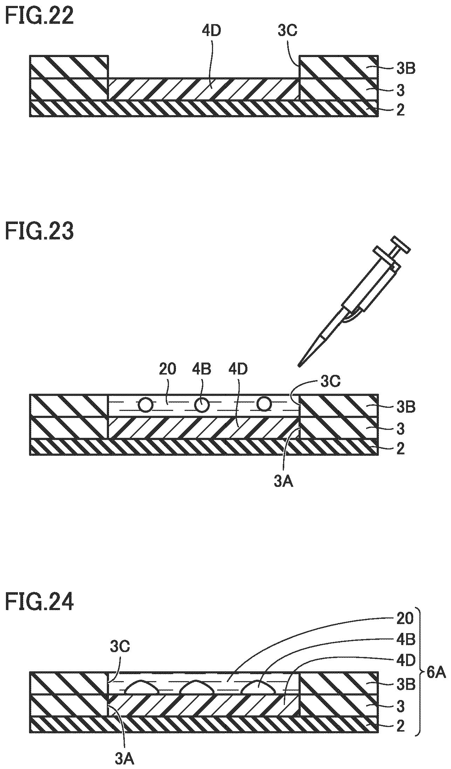

Fig. 21 is a schematic perspective view illustrating a second step of the supporting method in the example in which the gel supports the sample with the bonding. Fig. 22 is a schematic sectional view of a portion extending along XXII-XXII line in Fig. 21. Referring to Fig. 21 and Fig. 22, another frame 3B is installed on frame 3 illustrated in Fig. 20. Other frame 3B is preferably made in the same shape and substantially the same size and of the same material as the shape, the size, and the material of frame 3. A through-hole 3C is formed in other frame 3B. Through-hole 3C preferably has the same shape and substantially the same size as the shape and the size of through-hole 3A.

Fig. 23 is a schematic sectional view illustrating a third step of the supporting method in the example in which the gel supports the sample with the bonding. Referring to Fig. 23, sample 4B and a culture solution 20 in which sample 4B is suspended are supplied into through-hole 3C of frame 3B. Culture solution 20 is preferably supplied to fill the inside of through-hole 3C. Note that, when gel 4D is thinner than frame 3, parts of sample 4B and culture solution 20 may be disposed to be in a region in through-hole 3A.

Fig. 24 is a schematic sectional view illustrating a fourth step of the supporting method in the example in which the gel supports the sample with the bonding. Referring to Fig. 24, supplied sample 4B further falls compared with the supply time in Fig. 23 and is bonded on the upper surface of gel 4D. Accordingly, a gel sheet chip 6A including film 2, frame 3, other frame 3B, gel 4D on film 2, sample 4B bonded on gel 4D, and culture solution 20 filling the inside of through-hole 3C of other frame 3B is formed.

Note that in the example illustrated in Fig. 18 and Fig. 19, as in the example illustrated in Fig. 20 to Fig. 24, for example, a culture solution and a buffer solution for drying prevention may be supplied into through-hole 3A.

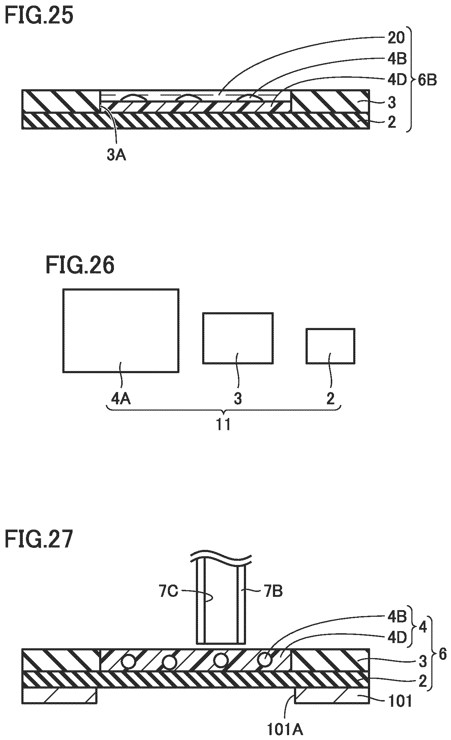

Fig. 25 is a schematic diagram illustrating a modification of Fig. 24. Referring to Fig. 25, the same treatment as the treatment illustrated in Fig. 23 and Fig. 24 is performed on gel 4D formed thinner than frame 3 as in Fig. 12 or Fig. 16. In Fig. 25, sample 4B is supported on the upper surface of gel 4D by bonding. In Fig. 25, culture solution 20 is supplied to immerse sample 4B. Accordingly, gel 4D, sample 4B, and culture solution 20 are filled in through-hole 3A. Fig. 25 illustrates generally the same configuration as the configuration illustrated in Fig. 24. However, in Fig. 25, a gel sheet chip 6B does not include frame 3B and includes only frame 3. All of gel 4D, sample 4B, and culture solution 20 are filled in through-hole 3A. Therefore, in Fig. 25, gel sheet chip 6B including film 2, frame 3, gel 4D on film 2, sample 4B bonded on gel 4D in through-hole 3A of frame 3, and culture solution 20 filling the inside of through-hole 3A of frame 3 is formed. Gel sheet chip 6B may have such a configuration.

As explained above, sample 4B being supported by gel 4D in the present embodiment includes not only the example in which sample 4B is embedded in gel 4D as illustrated in Fig. 11 but also the example in which sample 4B is bonded to the outer surface of gel 4D to thereby be fixed to gel 4D as illustrated in Fig. 24 and Fig. 25. According to both of the examples explained above, gel sheet 4 is formed by gel 4D and sample 4B.

Fig. 26 is a schematic diagram illustrating a sample collecting set for fixing a sample to be collected according to the first embodiment. Referring to Fig. 26, the sample collecting set may be implemented in a form of a sample collecting set 11 including the members used above, that is, gel material 4A (a drug) capable of, by forming gel, supporting sample 4B to be collected, film 2 on which the gel can be placed, and frame 3. Frame 3 can be placed on film 2 and through-hole 3A is formed in frame 3. That is, in sample collecting set 11, gel material 4A, film 2, and frame 3 are not integrated and are included as separate bodies. Sample collecting set 11 may further include other frame 3B in addition to the above. In sample collecting set 11, a user supports a sample using gel material 4A in a fluid state before being fixed. Sample collecting set 11 makes it possible to optionally manufacture gel sheet chip 6 and the like with high flexibility on the user side. If sample collecting set 11 is used, the sample collecting method explained above can be easily implemented on the user side.

### (Sample collecting method)

Fig. 27 is a schematic diagram illustrating a first step of a sample collecting method using a gel sheet chip in which gel supports a sample with embedding. Referring to Fig. 27, for example, gel sheet chip 6 illustrated in Fig. 7 and Fig. 11 is installed on XY stage for sample 101. At least a part of gel sheet 4 forming gel sheet chip 6 is disposed to overlap stage penetrating section 101A formed to penetrate XY stage for sample 101. In particular, sample 4B to be recovered in gel sheet 4 is disposed to overlap stage penetrating section 101A.

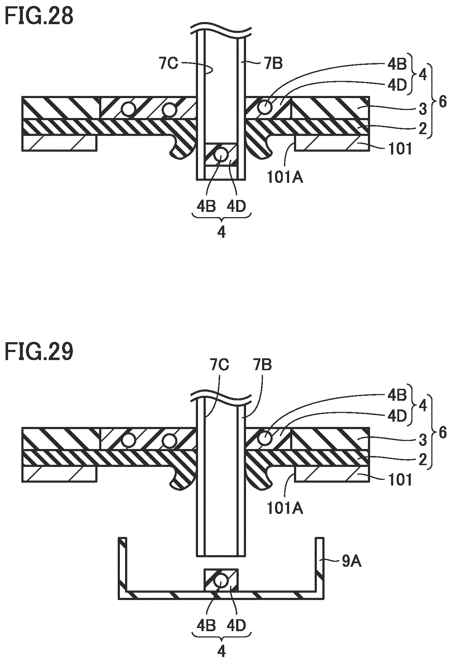

Fig. 28 is a schematic diagram illustrating a second step of the sample collecting method using the gel sheet chip in which the gel supports the sample with the embedding. Referring to Fig. 28, a needle for sample collection such as pin 7B falls with respect to a state illustrated in Fig. 27. A position to which pin 7B should be lowered is a position where target sample 4B to be collected is present as it is seen from an image acquired using an observation optical system (for example, observation optical system 106 illustrated in Fig. 4). At this time, in particular, gel sheet 4 of gel sheet chip 6 is punctured by pin 7B. That is, a portion where gel sheet 4 is punctured is damaged by passage of a sharp tip portion of pin 7B. Pin 7B penetrates gel sheet 4 while breaking gel sheet 4. A part of gel sheet 4 cut to be separated from original gel sheet 4 by pin 7B is a gel sheet portion 4E. Gel sheet portion 4E to be cut is preferably formed by gel 4D and sample 4B embedded on the inside of gel 4D. In pin 7B, hollow shaped section 7C is formed to generally penetrate pin 7B in a direction in which pin 7B extends. Cut gel sheet portion 4E is guided into hollow shaped section 7C and stored in hollow shaped section 7C.

Pin 7B further continues to fall from the state explained above. Pin 7B penetrates gel sheet portion 4E to be cut and a portion of film 2 disposed right under gel sheet portion 4E. Accordingly, film 2 is also damaged like gel sheet 4.

Fig. 29 is a schematic diagram illustrating a third step of the sample collecting method using the gel sheet chip in which the gel supports the sample with the embedding. Referring to Fig. 29, pin 7B further continues to fall from a state in which pin 7B penetrates gel sheet 4 and film 2 as illustrated in Fig. 28. At this time, pin 7B discharges gel sheet portion 4E in hollow shaped section 7C from the inside of hollow shaped section 7C. Discharged gel sheet portion 4E is recovered in container 9A.

The discharge of gel sheet portion 4E to the outside of hollow shaped section 7C is performed as follows. When at least the tip portion of pin 7B is lowered to a position where the tip portion reaches the inside of container 9A, air pressure is supplied into hollow shaped section 7C. The inside of hollow shaped section 7C of pin 7B is pressurized. For this reason, gel sheet portion 4E in hollow shaped section 7C is pressed downward and extruded to the outside of hollow shaped section 7C.

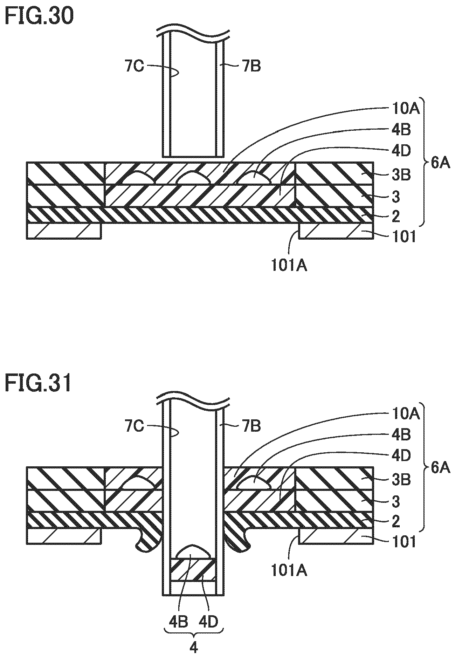

Fig. 30 is a schematic diagram illustrating a first step of a sample collecting method using a gel sheet chip in which gel supports a sample with bonding. Referring to Fig. 30, for example, gel sheet chip 6A illustrated in Fig. 24 is installed on XY stage for sample 101 and the same treatment as the treatment illustrated in Fig. 27 is basically performed.

Fig. 31 is a schematic diagram illustrating a second step of the sample collecting method using the gel sheet chip in which the gel supports the sample with the bonding. Referring to Fig. 31, the same treatment as the treatment illustrated in Fig. 28 is basically performed.

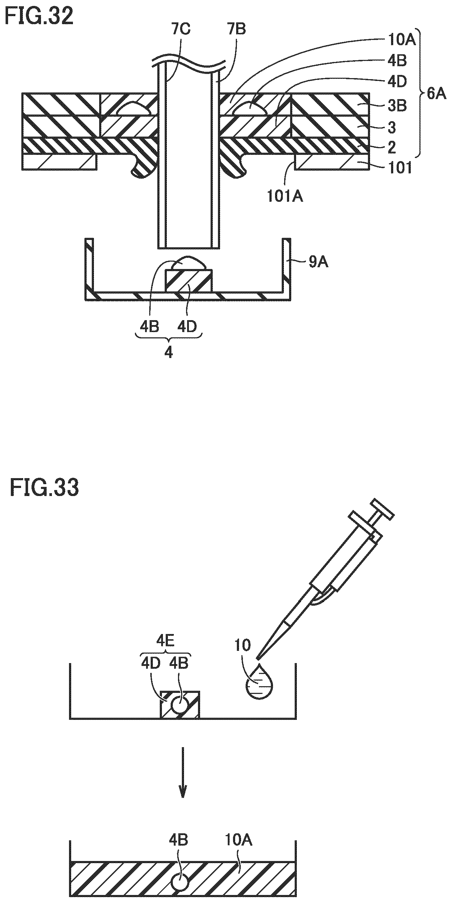

Fig. 32 is a schematic diagram illustrating a third step of the sample collecting method using the gel sheet chip in which the gel supports the sample with the bonding. Referring to Fig. 32, the same treatment as the treatment illustrated in Fig. 29 is basically performed.

### (Effects of the sample collecting method)

Since a floating cell cannot be bonded on a substrate, it is difficult to fix the cell in order to collect the cell. When a sample such as a cell cultured in a solid culture medium in a thin film culture apparatus is collected by suction, there is concern that collection accuracy is deteriorated.

Therefore, in the present embodiment, as explained above (Fig. 27 to Fig. 32), in the sample collecting method, a part of gel sheet 4 is cut by puncturing gel sheet 4 with pin 7B in which hollow shaped section 7C is formed and gel sheet portion 4E is stored in hollow shaped section 7C. At this time, pin 7B penetrates gel sheet 4 and a portion of film 2 right under gel sheet 4. Gel sheet portion 4E stored in hollow shaped section 7C by the penetration is recovered in container 9A.

With the sample collecting method explained above, sample 4B is supported by gel 4D of gel sheet 4 not to flow with respect to gel 4D. For this reason, it is possible to suppress collapse and scattering of sample 4B at the time when gel sheet portion 4E is stored in hollow shaped section 7C and when gel sheet portion 4E is collected. Since sample 4B is supported by gel 4D, it is possible to reduce the possibility of sample 4B moving in an unintended direction and damaging sample 4B. Accordingly, it is possible to perform sample collection with high stability, reproducibility, and position accuracy. For this reason, it is possible to collect sufficiently supported and fixed cell at higher accuracy compared with when a sample cultured in a solid culture medium is sucked and collected.

If, in particular, an animal cell is collected by contact using a solid needle, it is likely that the needle comes into contact with the cell to thereby damage the cell. Further, in a method for fractioning a cell using a liquid flow of a cell sorter, a microfluidic channel, and the like, there is a problem in that damage occurs in the fractioned cell because the liquid flow is fast. However, in the present embodiment, a method for storing gel sheet portion 4E in hollow shaped section 7C of pin 7B is used. When gel sheet 4 is cut and collected such that, in particular, only one cell is included in gel sheet 4, if a cell is supported by, for example, a sheet member (gel) by embedding, the cell, for example, embedded and supported by the gel is cut out. For this reason, it is possible to reduce the possibility of directly puncturing and damaging the cell.

Further, as illustrated in Fig. 29 and Fig. 32, in the sample collecting method explained above, in a step in which gel sheet portion 4E is recovered in a recovering section, only gel sheet portion 4E may be recovered in the recovering section. In general, when a cell bonded to a sheet member is cut out, the sheet member is also cut out together with the cell. For this reason, a problem can occur in that the material of the sheet member is mixed in the collected cell to make it difficult to accurately analyze the cell. However, according to the present embodiment, unlike the sheet member, film 2, which is a member different from gel sheet portion 4E, is not recovered in the recovering section. Therefore, it is possible to eliminate the possibility of the material of film 2 being mixed in sample 4B to be recovered and affecting the accuracy of an analysis of sample 4B.

### (Action effects)

In the present embodiment, the gel sheet chip to which the sample to be collected is fixed is disclosed. Gel sheet chip 6 includes film 2, frame 3, gel 4D, and sample 4B to be collected. Frame 3 is placed on film 2 and through-hole 3A is formed in frame 3. Gel 4D is disposed on film 2 in through-hole 3A. Sample 4B to be collected is supported by gel 4D.

Sample 4B is supported by gel 4D in gel sheet 4. Therefore, even if gel sheet 4 is penetrated by pin 7B illustrated in Fig. 27 to Fig. 32, sample 4B does not flow with respect to gel 4D. For this reason, it is possible to suppress collapse and scattering of sample 4B at the time when gel sheet portion 4E is stored in hollow shaped section 7C and when gel sheet portion 4E is collected. Since sample 4B is supported by gel 4D, it is possible to reduce the possibility of sample 4B moving in an unintended direction and damaging sample 4B when sample 4B is penetrated. Accordingly, it is possible to perform sample collection with high stability, reproducibility, and position accuracy. If gel 4D and film 2 are formed of a soft and easily damaged material, gel 4D and film 2 are easily damaged by penetration of pin 7B. For this reason, the step of cutting a part of gel sheet 4 is performed without difficulty.

As illustrated in Fig. 10 and Fig. 15, a flat plate (slide glass 5) is placed on frame 3. For this reason, the thickness of gel material 4A in through-hole 3A is controlled by pressing slide glass 5 downward. In other words, frame 3 controls the thickness of gel material 4A. Accordingly, sample 4B can be embedded without protruding or separating from gel 4D. Sample 4B is securely supported (embedded or bonded) by gel 4D. Positional deviation, scattering, and the like of sample 4B can be prevented. Accordingly, it is possible to perform sample collection with high stability, reproducibility, and position accuracy.

Further, gel 4D is preferably in contact with the inner wall surfaces that forms through-hole 3A of frame 3. Accordingly, there is an effect of preventing scattering and positional deviation of sample 4B supported by gel 4D (sample 4B is highly accurately fixed in a desired position).

If a gel sheet chip does not include frame 3 and includes only film 2 and gel sheet 4, it is difficult to handle fluid sample 4C. However, in the present embodiment, gel sheet chip 6 includes frame 3. It is easy to handle fluid sample 4C by bringing fluid sample 4C surrounded by frame 3 into contact with the inner wall surface of frame 3. As a result, positional deviation and the like of sample 4B included in gel 4D (gel sheet 4) is prevented.

In gel sheet chip 6 explained above, the thickness of gel 4D may be equal to or less than the thickness of frame 3. That is, the thickness of gel 4D may be substantially the same as the thickness of frame 3 (see Fig. 7 and Fig. 11). Gel 4D may be thinner than frame 3 (see Fig. 12 and Fig. 16). Because of the presence of frame 3, it is possible to freely control the thickness of gel 4D according to the shape and the dimension of slide glasses 5 and 5A at the time when gel sheet chip 6 is manufactured. By freely controlling the thickness of gel 4D, it is possible to cut gel sheet portion 4E (see Fig. 29 and Fig. 32) having any size.

In gel sheet chip 6 explained above, sample 4B may be disposed on the upper surface of gel 4D to adhere to gel 4D. Accordingly, for example, compared with when sample 4B is stored in gel 4D, gel sheet chip 6 has the following advantages. If sample 4B is a cell, by bonding a mixture of a cell having an adhesive ability and a cell not having an adhesive ability to gel 4D and then performing sample collecting operation, it is possible to selectively fraction only the cell adhering to gel 4D.

Gel sheet chip 6 may further include, on frame 3, other frame 3B placed to surround sample 4B on the upper surface of gel 4D. Accordingly, it is possible to form gel sheet chip 6 having a configuration in which, for example, gel 4D is filled in through-hole 3A of frame 3 on the lower side, sample 4B is disposed in through-hole 3C of frame 3B, and sample 4B adheres to the upper surface of gel 4D.

Conversely, in gel sheet chip 6, sample 4B may be disposed in gel 4D in through-hole 3A. Accordingly, it is possible to fix sample 4B irrespective of presence or absence of adhesion of sample 4B to gel 4D. It is possible to fraction any sample 4B out of gel 4D.

In gel sheet chip 6, sample 4B may be a cell or a cell aggregate. For example, if plurality of samples 4B are gathered in gel sheet 4 and form an aggregate, in particular, when the size of the aggregate is large, it is likely that sample 4B is punctured by pin 7B to damage sample 4B. If sample 4B is damaged, for example, it is sometimes difficult to analyze the cross section of a tissue of sample 4B. Therefore, plurality of samples 4B are preferably dispersed to be separated from one another. This makes it possible to suppress damage due to puncture into sample 4B. However, in particular, when the size of the aggregate is small and can pass through hollow shaped section 7C and the possibility of sample 4B being punctured by pin 7B is small, such a form may be adopted.

Note that, for example, in order to disperse plurality of samples 4B in gel sheet 4 to be disposed to be separated from one another as illustrated in Fig. 7, it is preferable that treatment for sufficiently dispersing low-concentration samples 4B in a gel solution is performed. Here, low concentration means concentration of 3.5×10⁴/mL or less, for example, in the case of a sample having a diameter of 10 µm.

In the above explanation, an example of sample 4B is a single (isolated) cell or an aggregate of a gathered plurality of cells. However, sample 4B may be a microorganism or a plant other than a cell. Sample 4B may be any one selected out of a group consisting of metal, plastic, protein, oil, and polysaccharide that are materials other than an organism.

In gel sheet chip 6, gel 4D may be made of a material having adhesion to a cell. Accordingly, sample 4B can be bonded and supported on the surface of gel 4D. When gel 4D embeds sample 4B, if gel 4D has adhesion, sample 4B is more securely supported by gel 4D.

Gel 4D is preferably made of a material having a characteristic of capable of being decomposed and dissolved. Fig. 33 is a schematic sectional view illustrating a first example of a step of dissolving gel from a gel sheet portion and recovering only a cell. Referring to Fig. 33, it is also possible to recover only a cell (sample 4B) by dissolving, from cut gel sheet portion 4E, gel 4D that supports and fixes sample 4B. Specifically, gel sheet portion 4E in which 5×10⁵ cells/mL of a fibroblast is disposed on the inside of gel 4D as sample 4B is stored on the inside of an implement. For example, an EDTA (ethylenediaminetetraacetic acid) solution having concentration of 1% is supplied to the inside of the implement as a gel dissolving agent 10. In Fig. 33, gel 4D is made of a mixture of alginic acid having a content ratio of 1.0% and collagen having content density of 1.2 mg/mL. Gel dissolving agent 10 dissolves gel 4D. Accordingly, as illustrated in a figure on the lower side of Fig. 33, gel dissolving agent 10 changes to a state including dissolved gel 4D and a state of being stayed in the implement as a gel dissolving agent 10A. Since gel dissolving agent 10A is in a liquid state, it is possible to recover only sample 4B by discarding gel dissolving agent 10A.

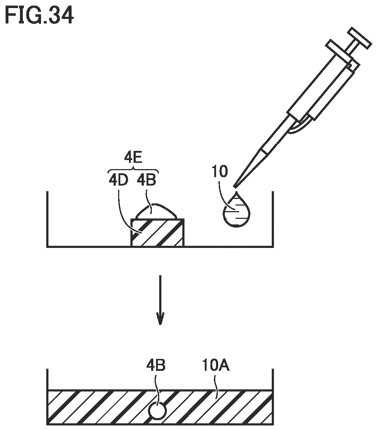

Fig. 34 is a schematic sectional view illustrating a second example of the step of dissolving the gel from the gel sheet portion and recovering only the cell. Referring to Fig. 34, sample 4B included in gel sheet portion 4E may be bonded to the outer side of gel 4D. In this case, as in Fig. 33, for example, an EDTA solution having concentration of 1% is supplied to the inside of the implement as gel dissolving agent 10.

Gel 4D is preferably made of a material capable of decomposing a cross-linking point and decomposing a gel material molecule. Accordingly, it is possible to decompose gel 4D from gel sheet portion 4E after cutting gel sheet portion 4E and extract only sample 4B. For example, it is assumed that sodium alginate is used as gel 4D. In this case, it is possible to dissolve alginate gel and obtain sample 4B by adding alginate lyase or a chelating agent (EDTA or the like). The alginate lyase is an alginic acid-degrading enzyme. The chelating agent removes calcium ions necessary for cross-linking of the alginate gel.

The alginate lyase is highly specific for alginic acid. The EDTA has main action of chelating calcium ions and magnesium ions in a culture medium. For this reason, a method using the alginate lyase and the EDTA is a method with low toxicity to a cell and low invasiveness in sample collection.

In the present embodiment, a manufacturing method for gel sheet chip 6 to which sample 4B to be collected is fixed is disclosed. In the manufacturing method for gel sheet chip 6, frame 3 in which through-hole 3A is formed is installed on film 2. Fluid sample 4C including sample 4B to be collected is supplied into through-hole 3A on film 2. The thickness of fluid sample 4C is uniformized by sandwiching the structure including film 2, frame 3, and fluid sample 4C with flat plates (slide glasses 1 and 5) installed on the upper side and the lower side of the structure. Fluid sample 4C is gelatinized. Accordingly, as explained above, the thickness of gel material 4A is controlled. It is possible to provide gel sheet chip 6 capable of collecting sample 4B with high stability, reproducibility, and position accuracy without damaging sample 4B.

### (Modifications)

The present embodiment explained above may have characteristics explained below. Note that characteristics of members explained below may be combined as appropriate.

First, from the viewpoint of suppressing a deficiency in which the shape of gel sheet portion 4E stored by the puncture by pin 7B is collapsed in, for example, hollow shaped section 7C, the inner wall surface of pin 7B may have, for example, at the tip portion of pin 7B, a taper shape extending in a direction inclined with respect to the direction in which pin 7B extends. In the taper shape, an angle with respect to the direction in which pin 7B extends may be, for example, 90° or less.

Second, the needle used in the present embodiment is not limited to pin 7B and only has to be any needle including a hollow shaped section. The needle may be, for example, an injection needle, a hollow shaped section of which has a columnar shape (that is, the entire needle has a cylindrical shape).

Third, from the viewpoint of easily discharging stored gel sheet portion 4E, hollow shaped section 7C used in the present embodiment is preferably coated with a material having low protein absorption and low affinity with gel 4D. Specifically, for example, a fluorine-based material may be coated on the inner wall surface of hollow shaped section 7C. Note that, here, gel 4D is, for example, an MPC polymer (2-methacryloyloxyethyl phosphorylcholine).

In the present embodiment, gel 4D is not limited to a generally used material such as collagen gel explained above and may be formed by any one selected out of a group consisting of tamarind seed gum, pectin, and carrageenan.

### (Second Embodiment)

### (Sample collecting method)

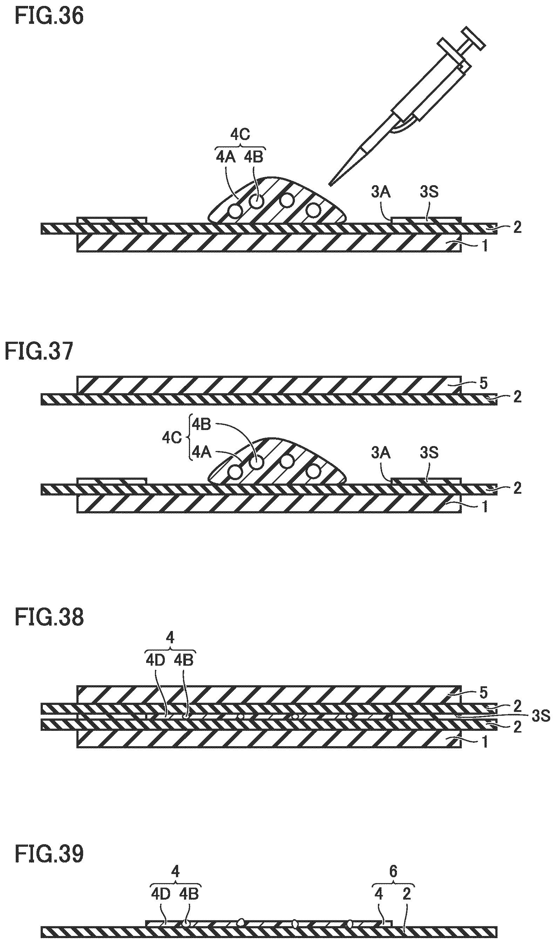

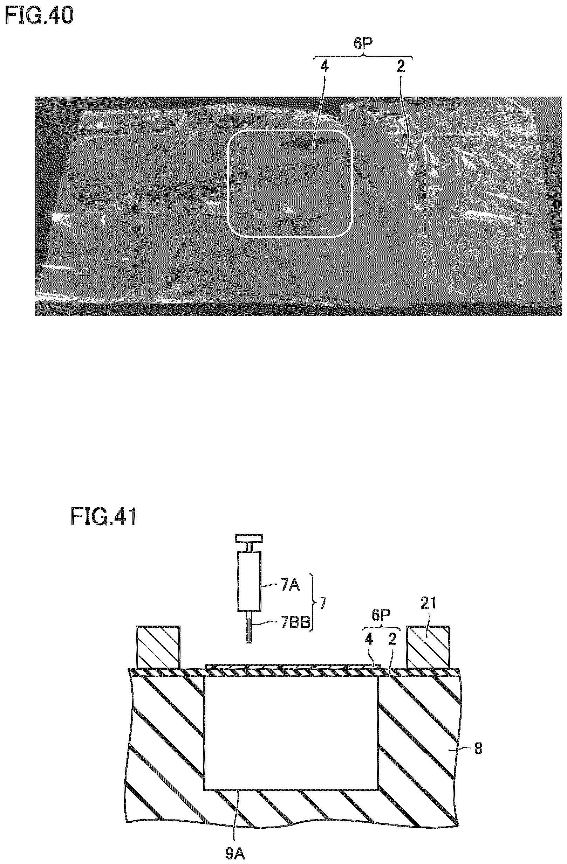

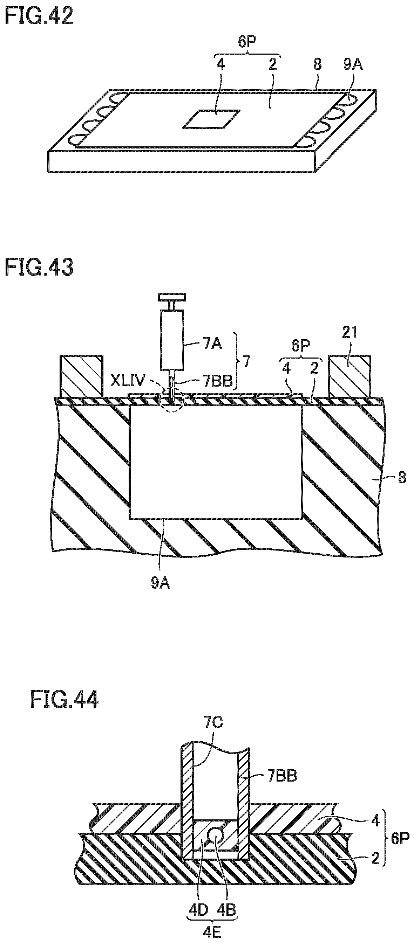

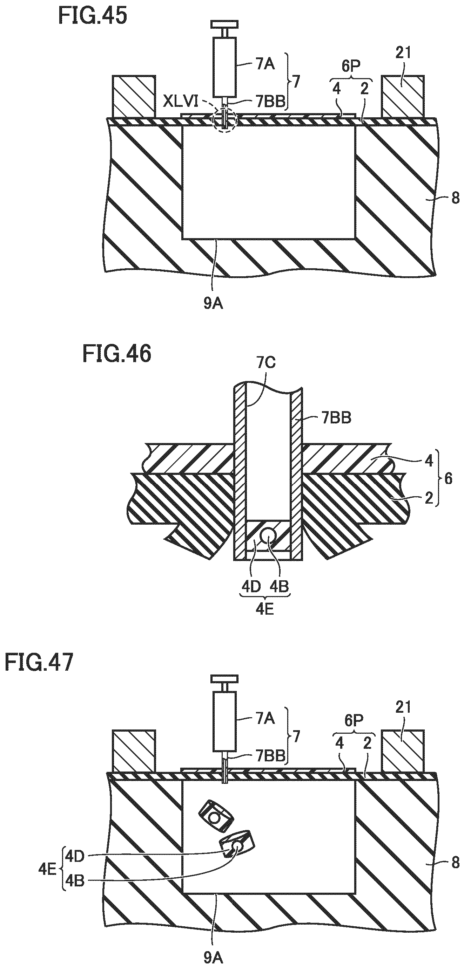

First, characteristics of the present embodiment are briefly explained. As illustrated in Fig. 39 and Fig. 40, gel sheet 4, in which sample 4B to be collected is supported by gel 4D, is placed on film 2. As illustrated in Fig. 44, gel sheet 4 on film 2 is punctured by an injection needle 7BB including hollow shaped section 7C to cut a part of gel sheet 4. Cut gel sheet portion 4E is stored in hollow shaped section 7C. As illustrated in Fig. 47, gel sheet portion 4E is collected by discharging gel sheet portion 4E in hollow shaped section 7C from hollow shaped section 7C. The embodiment is explained in detail below.

### (Overall configuration of a sample collecting apparatus)

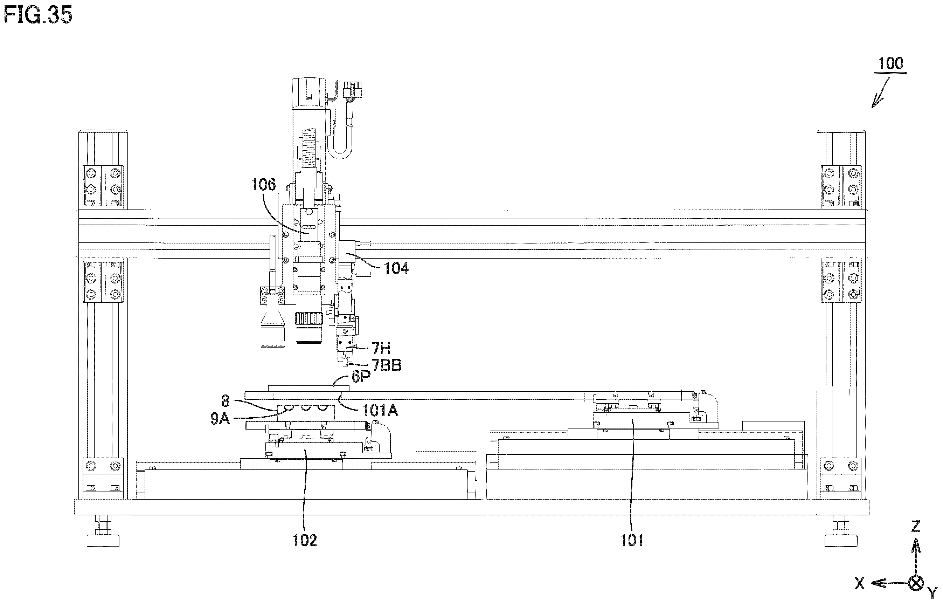

Fig. 35 is a schematic front view of the inside of a sample collecting apparatus according to the second embodiment. Note that, for convenience of explanation, an X direction, a Y direction, and a Z direction are introduced. Referring to Fig. 35, a sample collecting apparatus 100 is an apparatus for collecting a sample from a gel plate 6P serving as a sample explained below.

XY stage for container 102 fixes a plate 8 in which a container 9A capable of storing a part of gel plate 6P is formed. Accordingly, in Fig. 35, collecting mechanism 104, gel plate 6P, and plate 8 in which container 9A is formed are disposed in this order from the upper side to the lower side in the Z direction.

Since Fig. 35 is the same as Fig. 1 except those described above, the explanation referring to Fig. 35 is not repeated.

### (Configurations of the collecting mechanism and the collecting needle holder)

The second embodiment is the same as Fig. 2 and Fig. 3 in the first embodiment except that pin 7B, which is the collecting needle, illustrated in Fig. 2 and Fig. 3 is replaced with injection needle 7BB. Therefore, the explanation referring to Fig. 2 and Fig. 3 is not repeated.

In the following explanation, an implement playing a central role of collection including syringe 7A and injection needle 7BB is explained as an injector 7.

### (Details of a sample collecting method)

Fig. 36 is a schematic sectional view illustrating a first step of a sample collecting method according to the second embodiment. Referring to Fig. 36, film 2 is placed on, for example, slide glass 1. As an example, slide glass 1 is a thin plate material made of glass having thickness of approximately 1.2 mm. Film 2 is formed thin by a soft material to be easily damaged by puncture by a needle. As an example, film 2 is, for example, polyvinylidene chloride having thickness of approximately 10 µm. That is, as film 2, for example, a thin film for food packaging is preferably used. A spacer 3S is installed on film 2. Spacer 3S may be formed by the same glass material as a glass material forming slide glass 1. The thickness of spacer 3S may be 100 µm or more and 200 µm or less. For example, the thickness of spacer 3S may be 100 µm or may be 200 µm. In spacer 3S, through-hole 3A is formed in the center of spacer 3S when viewed from the upper side of Fig. 36 (in plan view). Through-hole 3A penetrates spacer 3S. For this reason, the inside of through-hole 3A is a hollow. A material such as glass forming spacer 3S is not disposed in through-hole 3A. A plane shape of through-hole 3A is any shape such as a rectangular shape.

Subsequently, fluid sample 4C having fluidity in which one or more samples 4B are included in gel material 4A having fluidity capable of becoming gel is supplied into through-hole 3A. Sample 4B is a cell to be collected or the like. In Fig. 36, plurality of samples 4B are disposed one by one at intervals from one another. Gel material 4A is, for example, 1.5% agarose gel obtained by adding a dye having fluorescence to, for example, 1.5 mL of a gel precursor. However, gel material 4A is not limited to this and may be, for example, collagen or may be a mixture of alginic acid and collagen.

Gel material 4A is a material that supports a cell, for example, with embedding. For this reason, gel material 4A is required to have biocompatibility. Accordingly, finally obtained gel is gel obtained by gelatinizing gel material 4A having biocompatibility. Here, having biocompatibility means that gel material 4A does not show toxicity such as inflammation even if a biological tissue such as a cell comes into contact with gel material 4A and has a nature with low invasiveness to the cell. Accordingly, it is possible to suppress damage to the cell (sample 4B) due to gel. It can be confirmed that a certain material has biocompatibility by the fact that the number of living cells does not decrease when cells are cultured on the certain material, living cells and dead cells are dyed, and inflammatory markers expressed by the cells or contents eluted from the cells are measured. Alternatively, it can be confirmed that the certain material has biocompatibility by the fact that the expression of the inflammatory markers has not increased or the contents have not eluted from the cells.

Gel material 4A is preferably transparent or semitransparent from the viewpoint of enabling observation of the position of embedded sample 4B. From the viewpoint of satisfying the above, gel material 4A is any one selected out of a group consisting of collagen, gelatin, fibrin, sodium alginate, gellan gum, agarose, hyaluronic acid, chitin, chitosan, polyethylene glycol, polyvinyl alcohol, and 2-methacryloyloxyethyl phosphorylcholine (MPC polymer).

Fig. 37 is a schematic sectional view illustrating a second step of the sample collecting method according to the second embodiment. Referring to Fig. 37, film 2 is pasted to the lower surface of slide glass 5. Slide glass 5 may be of the same material and size as slide glass 1 illustrated in Fig. 36. Film 2 on the upper side of fluid sample 4C illustrated in Fig. 37 may be of the same material and size as film 2 illustrated in Fig. 36.

Fig. 38 is a schematic sectional view illustrating a third step of the sample collecting method according to the second embodiment. Referring to Fig. 38, slide glass 5, to which film 2 is pasted, is placed on spacer 3S. Film 2 pasted to slide glass 5 is in contact with fluid sample 4C and spacer 3S. Slide glass 5 and film 2 press fluid sample 4C and spacer 3S downward. For this reason, fluid sample 4C and spacer 3S are sandwiched by slide glass 1 to which film 2 present under fluid sample 4C and spacer 3S is pasted and slide glass 5 to which film 2 present on fluid sample 4C and spacer 3S is pasted. Accordingly, slide glass 1, film 2, spacer 3S and fluid sample 4C, film 2, and slide glass 5 are stacked in this order from the lower side to the upper side.

Next, for example, when gel material 4A is a material that solidifies depending on temperature, the entire stacked components explained above is cooled to 4°C. Gel material 4A forming fluid sample 4C is changed to solidified gel 4D by the cooling. Gel sheet 4 in which sample 4B is supported on the inside of gel 4D is formed in through-hole 3A. Although gel 4D is solidified, gel 4D is soft enough to be easily damaged by puncture by a needle. Note that sample 4B being supported by gel 4D means, for example, a state in which the periphery of sample 4B is embedded to be covered and buried by hard gel 4D and sample 4B does not flow.

Fig. 39 is a schematic sectional view illustrating a fourth step of the sample collecting method according to the second embodiment. Fig. 40 is a schematic perspective view of a gel plate. Referring to Fig. 39 and Fig. 40, after gel sheet 4 is provided, film 2 and slide glass 5 placed on the upper side of gel sheet 4 are removed and slide glass 1 and spacer 3S are also removed. Accordingly, gel plate 6P including film 2 and gel sheet 4 placed on film 2 is formed. In gel sheet 4, sample 4B to be collected is supported by solidified gel 4D, for example, by embedding. Gel 4D is formed on film 2 in a sheet state. That is, gel sheet 4 includes gel 4D formed on film 2 in a sheet state and supporting sample 4B to be collected. In the present embodiment, "provide gel sheet 4" means that a step itself of supporting sample 4B in gel 4D with embedding or the like to obtain gel sheet 4 may be performed on film 2 or, for example, gel sheet 4 formed in advance on the outside may be attached on film 2 later. As a method for forming gel sheet 4, it is conceivable to prepare gel sheet 4 in advance on any flat surface. PBS (Phosphate-buffered saline) is dripped on gel plate 6P illustrated in Fig. 40 to prevent drying.

Fig. 41 is a schematic sectional view illustrating a fifth step of the sample collecting method according to the second embodiment. Fig. 42 is a schematic enlarged perspective view illustrating a plate and a gel plate on the plate included in Fig. 41. Referring to Fig. 41 and Fig. 42, formed gel plate 6P is punctured by injector 7 from above. Specifically, in the injector 7, for example, injection needle 7BB having thickness of 20 G is attached to 1 mL syringe 7A. In injection needle 7BB, hollow shaped section 7C is formed to generally penetrate injection needle 7BB in a direction in which injection needle 7BB extends. The inside of hollow shaped section 7C is a hollow. A material forming injection needle 7BB is not disposed in hollow shaped section 7C.

On the other hand, gel plate 6P is placed on plate 8. Plate 8 is a culture plate for cell culture. A plurality of containers 9A functioning as recovering sections are formed in plate 8. Plurality of containers 9A are depressed concave-shaped portions of the upper surface of plate 8. Since containers 9A are formed in the concave shape, a cut part (gel sheet portion 4E) of gel sheet 4 can be stored in containers 9A. As plurality of containers 9A, ninety-six containers 9A in total may be formed, for example, in four rows in the depth direction and twenty-four rows in the lateral direction in Fig. 42 at intervals from one another. A plane shape of container 9A is any shape such as a circular shape. In Fig. 41 and Fig. 42, film 2 forming gel plate 6P is in contact with the upper surface of plate 8 to close at least a part of plurality of containers 9A. Accordingly, a part (a region on the upper side) of plate 8 may be disposed in stage penetrating section 101A illustrated in Fig. 35.

For example, a sheet fixing member 21 may be placed above gel plate 6P. Sheet fixing member 21 suppresses positional deviation of gel plate 6P caused by force being applied to gel 4D from above at the time of a step of cutting gel sheet 4. As means for suppressing positional deviation of gel plate 6P, an adhesive film tape may be used instead of sheet fixing member 21.

Plate 8, in which containers 9A functioning as culture containers are formed, is formed by any material selected out of a group consisting of polystyrene, polypropylene, polycarbonate, and polyethylene terephthalate.

Fig. 43 is a schematic sectional view illustrating a sixth step of the sample collecting method according to the second embodiment. Fig. 44 is a schematic enlarged sectional view of a region XLIV surrounded by a dotted line in Fig. 43. Referring to Fig. 43 and Fig. 44, injector 7 falls with respect to a state illustrated in Fig. 41. A position to which injector 7 should be lowered is a position where target sample 4B to be collected is present as it is seen from an image acquired using an observation optical system (for example, observation optical system 106 illustrated in Fig. 36). At this time, in particular, gel sheet 4 of gel plate 6P is punctured by injection needle 7BB. That is, a portion where gel sheet 4 is punctured is damaged by passage of a sharp tip portion of injection needle 7BB. Injection needle 7BB penetrates gel sheet 4 while breaking gel sheet 4. A part of gel sheet 4 cut to be separated from original gel sheet 4 by injection needle 7BB is gel sheet portion 4E. Gel sheet portion 4E to be cut is preferably formed by gel 4D and sample 4B embedded on the inside of gel 4D. Cut gel sheet portion 4E is guided into hollow shaped section 7C and stored in hollow shaped section 7C.

Fig. 45 is a schematic sectional view illustrating a seventh step of the sample collecting method according to the second embodiment. Fig. 46 is a schematic enlarged sectional view of a region XLVI surrounded by a dotted line in Fig. 45. However, in Fig. 46, illustration of plate 8 is omitted. Referring to Fig. 45 and Fig. 46, injection needle 7BB further continues to fall from a state illustrated in Fig. 43 and Fig. 44. Injection needle 7BB penetrates gel sheet portion 4E to be cut and a portion of film 2 disposed right under gel sheet portion 4E. Accordingly, as illustrated in Fig. 46, film 2 is also damaged like gel sheet 4.

Fig. 47 is a schematic sectional view illustrating an eighth step of the sample collecting method according to the second embodiment. Referring to Fig. 47, injection needle 7BB further continues to fall from a state in which injection needle 7BB has penetrated gel sheet 4 and film 2 as illustrated in Fig. 45 and Fig. 46. At this time, injection needle 7BB discharges gel sheet portion 4E in hollow shaped section 7C from hollow shaped section 7C. Discharged gel sheet portion 4E is recovered in container 9A.

The discharge of gel sheet portion 4E to the outside of hollow shaped section 7C is performed as follows. When at least the tip portion of injection needle 7BB is lowered to a position where the tip portion reaches the inside of container 9A, a piston disposed in syringe 7A is pushed in downward. The inside of hollow shaped section 7C of injection needle 7BB is pressurized by an air pressure generated at this time. Accordingly, gel sheet portion 4E in hollow shaped section 7C is pressed downward and extruded to the outside of hollow shaped section 7C. Note that, at this time, an upper part of container 9A is closed by gel plate 6P and a space in container 9A is formed as a closed chamber. The closed chamber is preferably filled with PBS.

Since injection needle 7BB penetrates gel plate 6P, injection needle 7BB breaks through both of gel sheet 4 and film 2. However, as illustrated in Fig. 46, it is preferable that only gel sheet 4 is stored and film 2 is not stored in hollow shaped section 7C. As a result, as illustrated in Fig. 47, it is preferable that only gel sheet portion 4E is recovered in container 9A and film 2 is not recovered in container 9A.

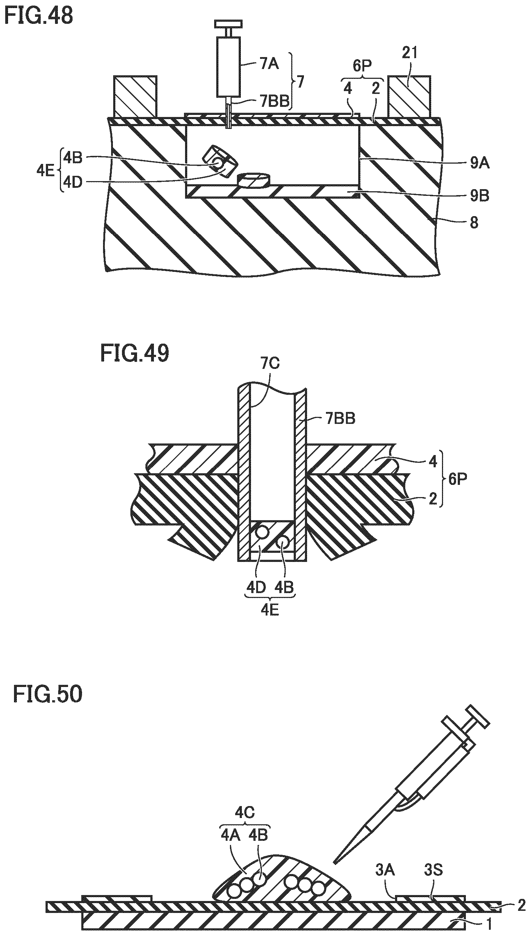

Fig. 48 is a schematic sectional view illustrating a first modification of the second embodiment. Referring to Fig. 48, since a form illustrated here is basically the same as the form illustrated in Fig. 47, the same components as the components illustrated in Fig. 47 are denoted by the same reference numerals and signs and explanation of the components is not repeated as long as functions and the like of the components are the same. However, in Fig. 48, a substrate 9B installed in container 9A is used as the recovering section. Gel sheet portion 4E discharged from the inside of hollow shaped section 7C is recovered on the upper surface of substrate 9B. In this regard, Fig. 48 is different in a configuration from Fig. 47 in which container 9A itself is the recovering section. Such a form may be adopted as long as the upper surface of substrate 9B is capable of receiving gel sheet portion 4E discharged from the inside of hollow shaped section 7C.

In the above explanation, for example, as illustrated in Fig. 39, plurality of samples 4B are included in gel sheet 4. As explained above, before gel sheet 4 is cut by injection needle 7BB and partially stored, plurality of samples 4B originally included in gel sheet 4 (in gel material 4A) may be dispersed to be disposed to be separated from one another. As a result, as illustrated in Fig. 46, only one sample 4B may be included in gel sheet portion 4E cut and stored in hollow shaped section 7C. However, in the present embodiment, sample 4B may be included as in the following modification.

Fig. 49 is a schematic sectional view illustrating a second modification of the second embodiment. Referring to Fig. 49, since a form illustrated here is basically the same as the form illustrated in Fig. 46, the same components as the components illustrated in Fig. 46 are denoted by the same reference numerals and signs and explanation of the components is not repeated as long as functions and the like of the components are the same. However, in Fig. 49, plurality of samples 4B are disposed one by one to be separated from one another in gel sheet portion 4E cut and stored in hollow shaped section 7C. In this regard, Fig. 49 is different from an example in which only one sample 4B is included in gel sheet portion 4E as illustrated in Fig. 46. In Fig. 49, two samples 4B are included in gel sheet portion 4E. However, the number of samples 4B included in a single gel sheet portion 4E is not limited to this and may be, for example, three or more.

In the present embodiment, isolated sample 4B is included in gel sheet portion 4E stored in hollow shaped section 7C by penetration of injection needle 7BB. Here, isolated means both of a case in which only one sample 4B is included in gel sheet portion 4E as illustrated in Fig. 46 to Fig. 48 and a case in which plurality of samples 4B are included one by one at intervals from one another as illustrated in Fig. 49.

Fig. 50 is a schematic sectional view illustrating a third modification of the second embodiment. Referring to Fig. 50, since a form illustrated here is basically the same as the form illustrated in Fig. 36, the same components as the components illustrated in Fig. 36 are denoted by the same reference numerals and signs and explanation of the components is not repeated as long as functions and the like of the components are the same. However, in Fig. 50, plurality of samples 4B are gathered and disposed as a single mass in gel material 4A forming fluid sample 4C. The mass of gathered plurality of samples 4B is referred to as aggregate. In Fig. 50, as an example, three samples 4B are gathered in the aggregate. However, not only this, but two or four or more samples 4B may form the aggregate.

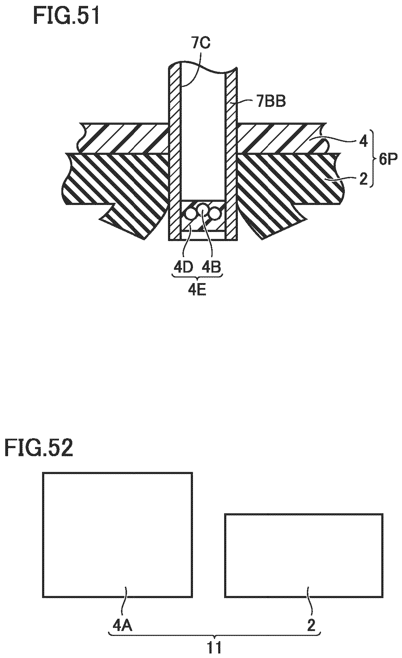

Fig. 51 is a schematic sectional view illustrating a step in which gel obtained from a fluid sample illustrated in Fig. 50 is cut. Referring to Fig. 51, since a form illustrated here is basically the same as the form illustrated in Fig. 46 and Fig. 49, the same components as the components illustrated in Fig. 46 and Fig. 49 are denoted by the same reference numerals and signs and explanation of the components is not repeated as long as functions and the like of the components are the same. However, in Fig. 51, plurality of samples 4B gathered as an aggregate are included in gel sheet portion 4E stored in hollow shaped section 7C. In this regard, Fig. 51 is different in a configuration from the figures referred to above. The aggregate needs to have size capable of passing through hollow shaped section 7C. That is, the aggregate is required to have size smaller than the width in a direction intersecting a direction in which hollow shaped section 7C extends.

Fig. 52 is a schematic diagram illustrating a sample collecting set for fixing a sample to be collected. Referring to Fig. 52, in the present embodiment, the sample collecting set may be implemented in a form of sample collecting set 11 including the members used in the above explanation, that is, gel material 4A (the drug) capable of, by forming gel, supporting sample 4B to be collected and film 2 on which the gel can be placed. That is, in sample collecting set 11, gel material 4A and film 2 are not integrated and are included as separate bodies. In sample collecting set 11, a user supports a sample using gel material 4A in a fluid state before being fixed. Sample collecting set 11 makes it possible to optionally manufacture gel plate 6P and the like with high flexibility on the user side. If sample collecting set 11 is used, it is possible to easily implement the sample collecting method explained above on the user side.

### (Action effects)

In the present embodiment, a sample collecting method for collecting a fixed sample to be collected is disclosed. In the sample collecting method, gel 4D supporting sample 4B to be collected is formed on film 2 in a sheet state and gel sheet 4 is provided on film 2. Gel sheet 4 on film 2 is punctured by a needle (injection needle 7BB) including hollow shaped section 7C, whereby a part of gel sheet 4 is cut and a cut portion (gel sheet portion 4E) of the gel sheet is stored in hollow shaped section 7C.

Gel sheet portion 4E in hollow shaped section 7C is discharged from hollow shaped section 7C, whereby gel sheet portion 4E is collected.

In general, when gel sheet 4 is cut and collected such that, in particular, only on cell is included in gel sheet 4, for example, if the cell is a cell that can be bonded to a sheet member (gel), the cell bonded and fixed to the sheet member is cut out. However, in the case of a cell non adhesive to the sheet member, the cell cannot be fixed to the sheet member. The cell is likely to collapse or is likely to collapse from the sheet member. In this case, it is difficult to accurately collect a desired cell.

Therefore, if a sample is collected as in the present embodiment, sample 4B is supported by gel 4D in gel sheet 4 (for example, fixed by embedding). Sample 4B does not flow with respect to gel sheet 4. For this reason, it is possible to suppress collapse and scattering of sample 4B at the time when gel sheet portion 4E is stored in hollow shaped section 7C and when gel sheet portion 4E is collected. Since sample 4B is supported by gel 4D, it is possible to reduce the possibility of sample 4B moving in an unintended direction and damaging sample 4B. Accordingly, it is possible to perform sample collection with high stability, reproducibility, and position accuracy. If gel 4D and film 2 are formed of a soft and easily damaged material, gel 4D and film 2 are easily damaged by penetration of injection needle 7BB. For this reason, the step of cutting a part of gel sheet 4 is performed without difficulty.

In the sample collecting method, in the step of collecting gel sheet portion 4E, gel sheet portion 4E cut by injection needle 7BB and a portion of film 2 disposed right under gel sheet portion 4E may be penetrated. After the penetrating step, gel sheet portion 4E may be recovered in the recovering section. Film 2 is used in gel plate 6P. For this reason, injection needle 7BB can cut and penetrate gel 4D and easily damage film 2 in contact with the lower side of gel 4D and penetrate film 2. Accordingly, it is possible to perform sample collection with high stability, reproducibility, and position accuracy.

In the sample collecting method explained above, the recovering section is substrate 9B or container 9A. The recovering section may be disposed right under injection needle 7BB. Accordingly, cut gel sheet portion 4E can be easily recovered.

In the sample collecting method explained above, in the step in which gel sheet portion 4E is recovered in the recovering section, only gel sheet portion 4E may be recovered in the recovering section. In general, when a cell bonded to a sheet member is cut out, the sheet member is cut out together with the cell. For this reason, a problem can occur in that the material of the sheet member is mixed in the collected cell to make it difficult to accurately analyze the cell. However, according to the present embodiment, unlike the sheet member, film 2, which is a member different from gel sheet portion 4E, is not recovered in the recovering section. Therefore, it is possible to eliminate the possibility of the material of film 2 being mixed in sample 4B to be recovered and affecting the accuracy of an analysis of sample 4B.

Specifically, it is preferable to adopt the following conditions in order to prevent torn film 2 from adhering to gel sheet portion 4E as illustrated in Fig. 46 and falling to the recovering section side. For example, a contact angle of water with respect to the surface of film 2 is preferably 60° or more. Accordingly, it is possible to easily peel film 2 from gel sheet 4. Alternatively, the thickness of film 2 is preferably 100 µm or less.

In the sample collecting method, gel sheet portion 4E may include gel 4D and sample 4B supported in gel 4D. Accordingly, sample 4B is securely fixed to gel 4D.

In the sample collecting method explained above, plurality of samples 4B may be included in gel sheet 4 and the sample collecting method may further include, before the step of storing gel sheet portion 4E in hollow shaped section 7C, a step of dispersing plurality of samples 4B in gel sheet 4 to be disposed to be separated from one another. Accordingly, isolated samples 4B may be included in gel sheet portion 4E stored in hollow shaped section 7C in the storing step.

For example, if plurality of samples 4B are gathered in gel sheet 4 to form an aggregate, in particular, when the size of the aggregate is large, it is likely that sample 4B is punctured by injection needle 7BB and damaged. If sample 4B is damaged, it is sometimes difficult to analyze the cross section of a tissue of sample 4B. Therefore, it is preferable to disperse plurality of samples 4B to be separated from one another as explained above. Accordingly, it is possible to suppress damage by puncture through sample 4B.

Specifically, in order to disperse plurality of samples 4B in gel sheet 4 to be disposed to be separated from one another, for example, as illustrated in Fig. 39, it is preferable that treatment for sufficiently dispersing low-concentration samples 4B in a gel solution is performed. Here, low concentration means concentration of 3.5×10⁴/mL or less, for example, in the case of a sample having a diameter of 10 µm.

However, in the present embodiment, gel sheet portion 4E stored in hollow shaped section 7C in the step of storing gel sheet portion 4E in hollow shaped section 7C may include an aggregate of samples 4B formed by gathering plurality of samples 4B. In particular, if the size of the aggregate is small and the aggregate can pass through hollow shaped section 7C and the possibility of sample 4B being punctured by injection needle 7BB is small, such a form may be adopted.

In the above explanation, an example of sample 4B is a single (isolated) cell or an aggregate of a gathered plurality of cells. However, sample 4B may be a microorganism or a plant other than a cell. Sample 4B may be any one selected out of a group consisting of metal, plastic, protein, oil, and polysaccharide that are materials other than an organism.

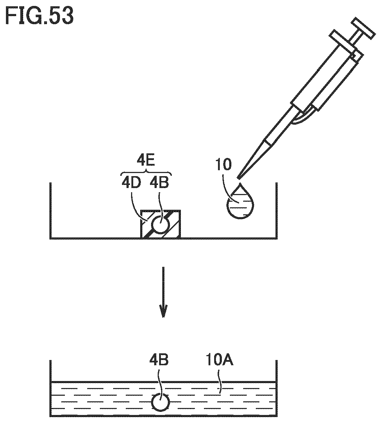

Fig. 53 is a schematic sectional view illustrating an example of a step of dissolving gel from a gel sheet portion and recovering only a cell. Referring to Fig. 53, it is also possible to recover only a cell (sample 4B) by dissolving, from cut gel sheet portion 4E, gel 4D that supports and fixes sample 4B. Specifically, gel sheet portion 4E in which 5×10⁵ cells/mL of a fibroblast is disposed on the inside of gel 4D as sample 4B is stored on the inside of an implement. For example, an EDTA (ethylenediaminetetraacetic acid) solution having concentration of 1% is supplied to the inside of the implement as gel dissolving agent 10. In Fig. 53, gel 4D is made of a mixture of alginic acid having a content ratio of 1.0% and collagen having content density of 1.2 mg/mL. Gel dissolving agent 10 dissolves gel 4D. Accordingly, as illustrated in a figure on the lower side of Fig. 53, gel dissolving agent 10 changes to a state including dissolved gel 4D and a state of being accumulated in the implement as gel dissolving agent 10A. Since gel dissolving agent 10A is in a liquid state, it is possible to recover only sample 4B by discarding gel dissolving agent 10A.

EDTA used as gel dissolving agent 10 chelates a divalent ion such as a calcium ion. For this reason, the EDTA is considered to have low toxicity to a cell. For gel 4D of alginic acid, an enzyme that decomposes gel 4D such as alginate lyase may be used as gel dissolving agent 10A. Even if the enzyme that decomposes gel 4D such as alginate lyase is used, it is possible to recover only a cell in a state in which invasion to the cell is small.

From the above explanation, the sample collecting method according to the present embodiment explained above may further include a step of dissolving gel 4D from gel sheet portion 4E and a step of extracting sample 4B remaining after the dissolving step. By using a mechanism that unlinks a cross-linked structure of gel 4D and a substance such as an enzyme that selectively decomposes gel 4D, it is possible to recover only the cell from recovered gel sheet portion 4E without damaging the cell.

### (Modifications)

The present embodiment explained above may have characteristics explained below. Note that characteristics of members explained below may be combined as appropriate.

First, "film 2" in the present embodiment is preferably a material that is soft and fragile enough to be easily damaged by pressing as small as falling of injection needle 7BB and enable injection needle 7BB to penetrate the material but is not greatly torn in a portion other than a punctured portion by injection needle 7BB and can maintain a state before puncture to a degree capable of supporting gel sheet 4 even after the penetration. From this viewpoint, film 2 is not limited to a thin film for food packaging such as polyvinylidene chloride and may be formed by, for example, a material explained below. That is, film 2 may be formed by a plastic film such as polyethylene or polyvinyl chloride. Alternatively, film 2 may be formed by polydimethylsiloxane or butyl rubber. From the viewpoint of making the punctured portion more easily damaged by the falling of injection needle 7BB to prevent damage to a portion other than the punctured portion, the thickness of film 2 is preferably 10 µm or more and 100 µm or less. From the viewpoint explained above, a maximum elongation rate (an extreme elongation rate) of film 2 is preferably 100% or more and 1300% or less. Further, in order to visually recognize the position of sample 4B through film 2, film 2 is preferably transparent or semitransparent suitable for observation by an optical system.

Second, from the viewpoint of suppressing a deficiency in which the shape of gel sheet portion 4E stored by injection needle 7BB with puncture is collapsed in, for example, hollow shaped section 7C, the inner wall surface of injection needle 7BB may have, for example, at the tip portion of injection needle 7BB, a taper shape extending in a direction inclined with respect to the direction in which injection needle 7BB extends. In the taper shape, an angle with respect to the direction in which injection needle 7BB extends may be, for example, 90° or less.

Third, the needle used in the present embodiment is not limited to injection needle 7BB and only has to be any needle including a hollow shaped section. The needle may be, for example, a pin, a hollow shaped section of which has a columnar shape (that is, the entire needle has a cylindrical shape).

Fourth, from the viewpoint of easily discharging stored gel sheet portion 4E, hollow shaped section 7C used in the present embodiment is preferably coated with a material having low protein absorption and low affinity with gel 4D. Specifically, for example, a fluorine-based material may be coated on the inner wall surface of hollow shaped section 7C. Note that, here, gel 4D is, for example, an MPC polymer (2-methacryloyloxyethyl phosphorylcholine).

In the present embodiment, gel 4D is not limited to a generally used material such as collagen gel explained above and may be formed by any one selected out of a group consisting of tamarind seed gum, pectin, and carrageenan.

### (Third Embodiment)

### (Material supplying method)

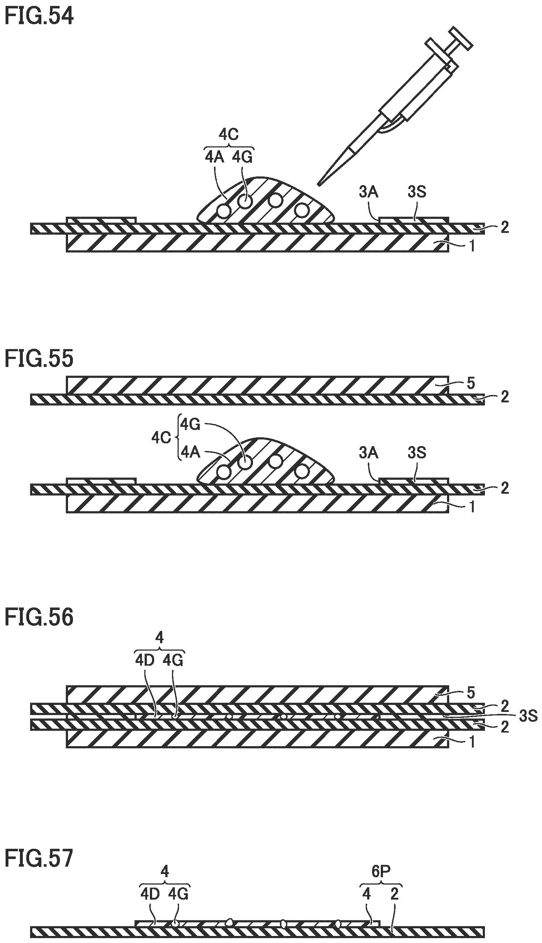

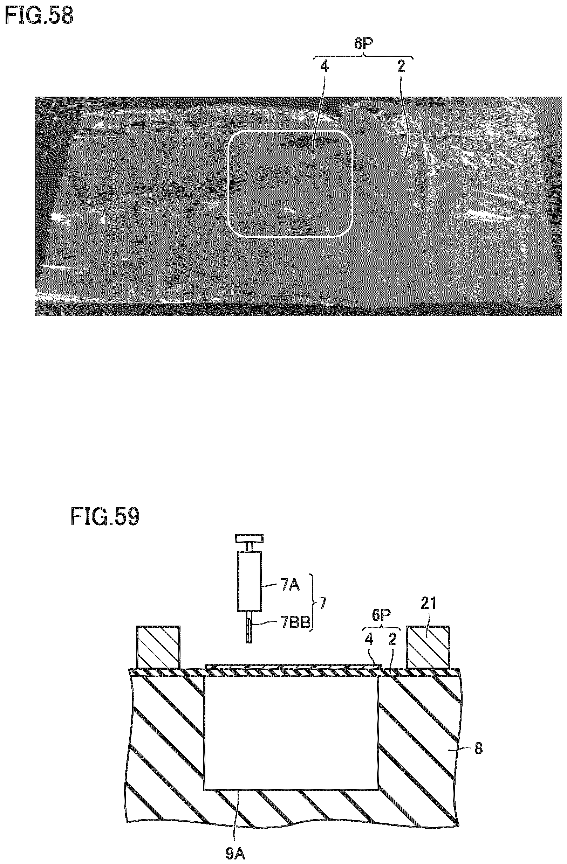

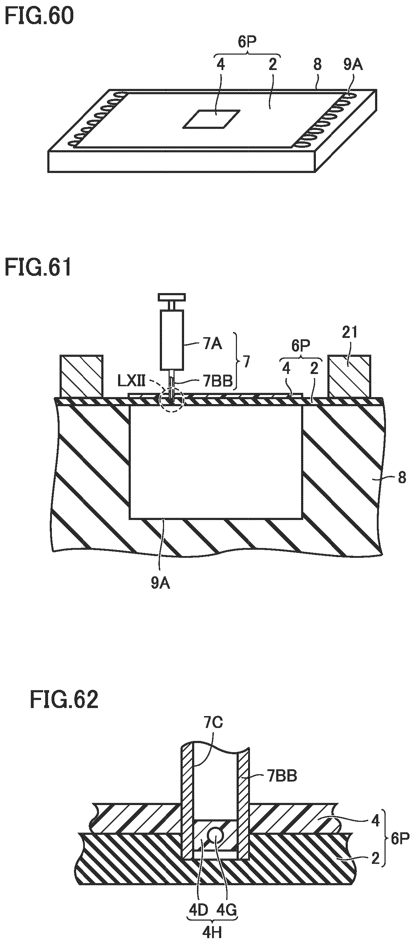

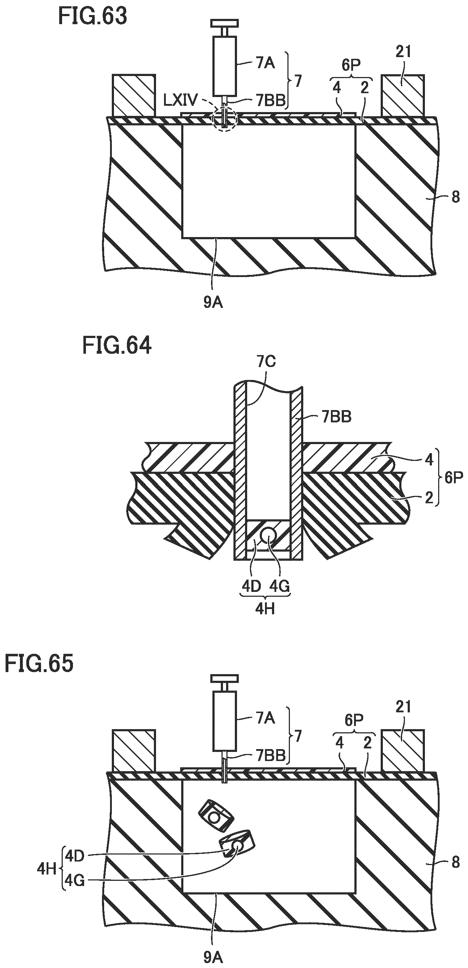

First, characteristics of the present embodiment are briefly explained. As illustrated in Fig. 57 and Fig. 58, gel 4D containing a factor 4G to be supplied is formed on film 2 in a sheet state and gel sheet 4 is provided on film 2. As illustrated in Fig. 62, gel sheet 4 on film 2 is punctured by injection needle 7BB including hollow shaped section 7C to cut a part of gel sheet 4. Solid gel 4H, which is a cut gel sheet portion, is stored in hollow shaped section 7C. As illustrated in Fig. 65, solid gel 4H in hollow shaped section 7C is discharged from hollow shaped section 7C, whereby solid gel 4H is supplied into, as a sustained-release material including factor 4G, container 9A in which a cell is cultured. The present embodiment is explained in detail below.

### (Overall configuration of a sample collecting apparatus)

In the present embodiment, the same sample collecting apparatus 100 as sample collecting apparatus 100 illustrated in Fig. 35 collects a sustained-release material from gel plate 6P serving as a sample explained below and supplies the sustained-release material. Since a configuration of sample collecting apparatus 100 is the same as the configuration illustrated in Fig. 35, explanation of the configuration is not repeated.

### (Configurations of a collecting mechanism and a collecting needle holder)

In the third embodiment, configurations of a collecting mechanism and a collecting needle holder are the same as the configurations in the second embodiment. Therefore, explanation of the configurations is not repeated.

### (Details of a sample collecting method)

Fig. 54 is a schematic sectional view illustrating a first step of a sample collecting method according to the third embodiment. Referring to Fig. 54, film 2 is placed on, for example, slide glass 1. As an example, slide glass 1 is a thin plate material made of glass having thickness of approximately 1.2 mm. Film 2 is formed thin by a soft material to be easily damaged by puncture by a needle. As an example, film 2 is, for example, polyvinylidene chloride having thickness of approximately 10 µm. That is, as film 2, for example, a thin film for food packaging is preferably used. Spacer 3S is installed on film 2. Spacer 3S may be formed by the same glass material as a glass material forming slide glass 1. The thickness of spacer 3S may be 100 µm or more and 200 µm or less. For example, the thickness of spacer 3S may be 100 µm or may be 200 µm. In spacer 3S, through-hole 3A is formed in the center of spacer 3S when viewed from the upper side of Fig. 54 (in plan view). Through-hole 3A penetrates spacer 3S. For this reason, the inside of through-hole 3A is a hollow. A material such as glass forming spacer 3S is not disposed in through-hole 3A. A plane shape of through-hole 3A is any shape such as a rectangular shape.

Next, fluid sample 4C having fluidity in which one or more factors 4G are included in gel material 4A having fluidity capable of becoming gel is supplied into through-hole 3A. Factors 4G are factors such as a drug or protein that affect a behavior of a cell. More specifically, factors 4G are growth factors or the like that accelerate growth of the cell. In Fig. 54, factors 4G are indicated by circles one by one from the viewpoint of easily imaging factors 4G. However, actually, factors 4G are not limited to such a shape. For example, factors 4G may be integrated as a whole. In any case, factors 4G are contained on the inside of gel material 4A.

Gel material 4A is, for example, 1.5% agarose gel obtained by adding a dye having fluorescence (a fluorescent pen cartridge) to, for example, 1.5 mL of a gel precursor. However, gel material 4A is not limited to this and may be, for example, collagen or may be a mixture of alginic acid and collagen. By mixing the dye having fluorescence in gel material 4A in advance, it is possible to perform a shape observation using a fluorescence microscope such as an all-field microscope or a confocal laser microscope.

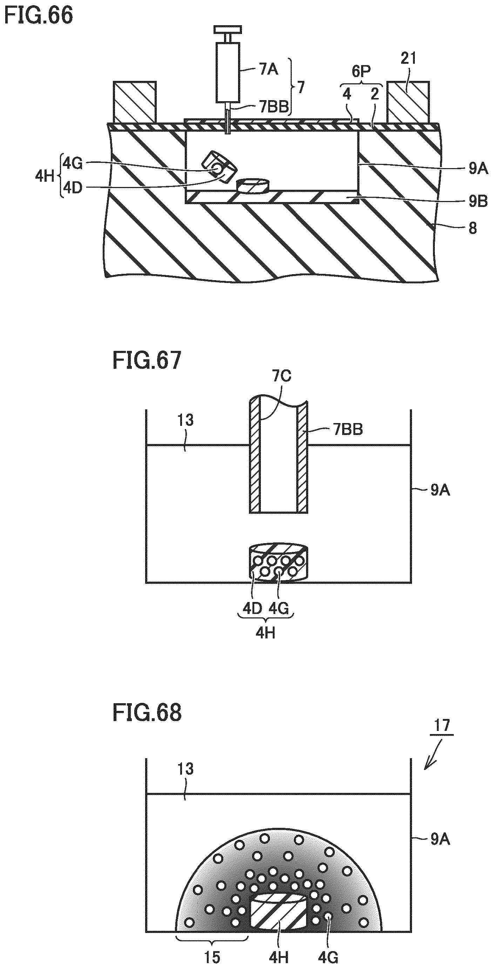

Factor 4G contained in gel material 4A is preferably any one selected out of a group consisting of protein, polysaccharide, and a low molecular weight compound. Specifically, the protein of factor 4G is low molecular weight protein such as cytokine. The polysaccharide of factor 4G is, for example, dextran. If the protein, the polysaccharide, and the low molecular weight compound are used, factor 4G contained in solid gel 4H (see Fig. 67) is sustainedly released, that is, gradually emitted, in a medium, from solid gel 4H explained below obtained by solidifying gel material 4A, whereby a concentration gradient of factor 4G can be formed.

Speed of factor 4G being sustainedly released from solid gel 4H explained below (see Fig. 67; the same applies below) can be controlled according to natures of gel 4D such as the concentration of gel 4D in solid gel 4H (gel material 4A), an amount of cross-linking points of gel 4D, and whether a material forming gel 4D is hydrophobic or hydrophilic. For example, when gel 4D is formed from polyacrylamide, by increasing the concentration of gel 4D from 0.5% to 2%, it is possible to reduce the speed of factor 4G being sustainedly released. A network of gel 4D is made finer by increasing the amount of the cross-linking points of gel 4D in solid gel 4H, whereby it is possible to reduce the speed of factor 4G being sustainedly released. If a molecule amount of factor 4G is increased, since transmission from meshes of gel 4D is inhibited, it is possible to reduce the speed of factor 4G being sustainedly released. Conversely, if the molecule amount of factor 4G is reduced, it is possible to increase the speed of factor 4G being sustainedly released. In this way, according to the molecule amount of contained factor 4G, it is possible to control the speed of factor 4G being sustainedly released.

Agarose is likely to degenerate protein or polysaccharide contained therein with heating at the time of dissolution. For this reason, for the agarose gel serving as gel 4D, it is preferable to use low-melting point agarose capable of dissolving at temperature lower than the degeneration temperature of protein or polysaccharide.

When solid gel 4H is formed using gel 4D that chemically cross-links amino acid residues, if protein such as a growth factor is contained in solid gel 4H, since a cross-linking point and factor 4G are cross-linked, a sustained release function is likely to be deactivated. For this reason, it is preferable that gel 4D having a gelatinizing mechanism not cross-linked with protein or the like is used.

From the above, gel material 4A is required to have biocompatibility. Accordingly, finally obtained gel is gel obtained by gelatinizing gel material 4A having biocompatibility. Here, having biocompatibility means that gel material 4A does not show toxicity such as inflammation even if a biological tissue such as a cell comes into contact with gel material 4A and has a nature with low invasiveness to the cell. Accordingly, it is possible to suppress damage to the cell due to the gel. It can be confirmed that a certain material has biocompatibility by the fact that the number of living cells does not decrease when cells are cultured on the certain material, living cells and dead cells are dyed, and inflammatory markers expressed by the cells or contents eluted from the cells are measured. Alternatively, it can be confirmed that the certain material has biocompatibility by the fact that the expression of the inflammatory markers has not increased or the contents have not eluted from the cells.

Gel material 4A is preferably transparent or semitransparent from the viewpoint of enabling observation of the position of contained factor 4G. From the viewpoint of satisfying the above, gel material 4A may be protein such as collagen, atelocollagen, gelatin, fibrin, or Matrigel. Alternatively, gel material 4A may be polysaccharide such as hyaluronic acid, agarose, alginic acid, chitin, or chitosan. Alternatively, gel material 4A may be a polymeric compound such as polyethylene glycol, polyvinyl alcohol, or polyacrylamide. Gel material 4A is not limited to polyacrylamide and acrylamide may be used. Gel material 4A may be a dielectric of these substances. Gel material 4A includes (consists of) at least type of a substance selected from the types described above.

Fig. 55 is a schematic sectional view illustrating a second step of the sample collecting method according to the third embodiment. Referring to Fig. 55, film 2 is pasted to the lower surface of slide glass 5. Slide glass 5 may be of the same material and size as slide glass 1 illustrated in Fig. 54. Film 2 on the upper side of fluid sample 4C illustrated in Fig. 55 may be of the same material and size as film 2 illustrated in Fig. 54.

Fig. 56 is a schematic sectional view illustrating a third step of the sample collecting method according to the third embodiment. Referring to Fig. 56, slide glass 5, to which film 2 is pasted, is placed on spacer 3S. Film 2 pasted to slide glass 5 is in contact with fluid sample 4C and spacer 3 S. Slide glass 5 and film 2 press fluid sample 4C and spacer 3S downward. For this reason, fluid sample 4C and spacer 3S are sandwiched by slide glass 1 to which film 2 present under fluid sample 4C and spacer 3S is pasted and slide glass 5 to which film 2 present on fluid sample 4C and spacer 3S is pasted. Accordingly, slide glass 1, film 2, spacer 3S and fluid sample 4C, film 2, and slide glass 5 are stacked in this order from the lower side to the upper side.

Next, for example, when gel material 4A is a material solidified by cooling, the entire stacked components explained above is cooled to 4°C. Gel material 4A solidified by cooling is, for example, agarose or gelatine. Alternatively, when gel material 4A is a material solidified by a little heating, the entire stacked components explained above is heated to 37°C. Gel material 4A solidified at 37°C is, for example, collagen. Gel material 4A forming fluid sample 4C is changed to solidified gel 4D by the cooling or the heating. Gel sheet 4 in which factor 4G is contained on the inside of gel 4D is formed in through-hole 3A. Although gel 4D is solidified, gel 4D is soft enough to be easily damaged by puncture by a needle.

Fig. 57 is a schematic sectional view illustrating a fourth step of the sample collecting method according to the third embodiment. Fig. 58 is a schematic perspective view of a gel plate. Referring to Fig. 57 and Fig. 58, after gel sheet 4 is provided, film 2 and slide glass 5 placed on the upper side of gel sheet 4 are removed and slide glass 1 and spacer 3S are also removed. Accordingly, gel plate 6P including film 2 and gel sheet 4 placed on film 2 is formed. Gel 4D is formed on film 2 in a sheet state. That is, gel sheet 4 includes gel 4D formed on film 2 in a sheet state and containing factor 4G to be supplied. In the present embodiment, "provide gel sheet 4" means that a step itself of containing factor 4G in gel 4D to obtain gel sheet 4 may be performed on film 2 or, for example, gel sheet 4 formed in advance on the outside may be attached on film 2 later. As a method for forming gel sheet 4, it is conceivable to prepare gel sheet 4 in advance on any flat surface. PBS (Phosphate-buffered saline) or a culture solution is dripped on gel plate 6P illustrated in Fig. 58 to prevent drying.

Fig. 59 is a schematic sectional view illustrating a fifth step of the sample collecting method according to the third embodiment. Fig. 60 is a schematic enlarged perspective view illustrating a plate and a gel plate on the plate included in Fig. 59. Referring to Fig. 59 and Fig. 60, formed gel plate 6P is punctured by injector 7 from above. Specifically, in the injector 7, for example, injection needle 7BB having thickness of 20 G is attached to 1 mL syringe 7A. In injection needle 7BB, hollow shaped section 7C is formed to generally penetrate injection needle 7BB in the direction in which injection needle 7BB extends. The inside of hollow shaped section 7C is a hollow. A material forming injection needle 7BB is not disposed in hollow shaped section 7C. In hollow shaped section 7C of injector 7BB having the thickness of 20G, a cross section intersecting a direction in which hollow shaped section 7C extends has, for example, a circular shape. The diameter of the circular shape is approximately 600 µm.

On the other hand, gel plate 6P is placed on plate 8. Plate 8 is a culture plate for cell culture. Plurality of containers 9A functioning as recovering sections are formed in plate 8. Plurality of containers 9A are depressed concave-shaped portions of the upper surface of plate 8. Since containers 9A are formed in the concave shape, a cut part (solid gel 4H) of gel sheet 4 can be stored in containers 9A. As plurality of containers 9A, ninety-six containers 9A in total may be formed, for example, in eight rows in the depth direction and twelve rows in Fig. 60 in the lateral direction at intervals from one another. A plane shape of container 9A is any shape such as a circular shape. In Fig. 59 and Fig. 60, film 2 forming gel plate 6P is in contact with the upper surface of plate 8 to close at least a part of plurality of containers 9A. Accordingly, a part (a region on the upper side) of plate 8 may be disposed in stage penetrating section 101A illustrated in Fig. 1.

Sheet fixing member 21 may be placed above gel plate 6P. Sheet fixing member 21 suppresses positional deviation of gel plate 6P caused by force being applied to gel 4D from above at the time of a step of cutting gel sheet 4. As means for suppressing positional deviation of gel plate 6P, an adhesive film tape may be used instead of sheet fixing member 21.

Plate 8, in which containers 9A functioning as culture containers are formed, is formed by any material selected out of a group consisting of polystyrene, polypropylene, polycarbonate, polyethylene terephthalate, and glass.

Fig. 61 is a schematic sectional view illustrating a sixth step of the sample collecting method according to the third embodiment. Fig. 62 is a schematic enlarged sectional view of a region LXII surrounded by a dotted line in Fig. 61. Referring to Fig. 61 and Fig. 62, injector 7 falls with respect to a state illustrated in Fig. 59. In particular, gel sheet 4 of gel plate 6P is punctured by injection needle 7BB. That is, a portion where gel sheet 4 is punctured is damaged by passage of a sharp tip portion of injection needle 7BB. Injection needle 7BB penetrates gel sheet 4 while breaking gel sheet 4. A part of gel sheet 4 cut to be separated from original gel sheet 4 by injection needle 7BB is solid gel 4H. Solid gel 4H to be cut is preferably formed by gel 4D and factor 4G contained on the inside of gel 4D. Cut solid gel 4H is guided into hollow shaped section 7C and stored in hollow shaped section 7C.

Fig. 63 is a schematic sectional view illustrating a seventh step of the sample collecting method according to the third embodiment. Fig. 64 is a schematic enlarged sectional view of a region LXIV surrounded by a dotted line in Fig. 63. However, in Fig. 64, illustration of plate 8 is omitted. Referring to Fig. 63 and Fig. 64, injection needle 7BB further continues to fall from a state illustrated in Fig. 61 and Fig. 62. Injection needle 7BB penetrates a gel sheet portion (solid gel 4H) to be cut and a portion of film 2 disposed right under solid gel 4H. Accordingly, as illustrated in Fig. 64, film 2 is also damaged like gel sheet 4.

Fig. 65 is a schematic sectional view illustrating an eighth step of the sample collecting method according to the third embodiment. Referring to Fig. 65, injection needle 7BB further continues to fall from a state in which injection needle 7BB has penetrated gel sheet 4 and film 2 as illustrated in Fig. 63 and Fig. 64. At this time, injection needle 7BB discharges solid gel 4H in hollow shaped section 7C from hollow shaped section 7C. Discharged solid gel 4H is supplied into, as a sustained-release material including factor 4G, container 9A in which a cell is cultured.

The discharge of solid gel 4H to the outside of hollow shaped section 7C is performed as follows. When at least the tip portion of injection needle 7BB is lowered to a position where the tip portion reaches the inside of container 9A, a piston disposed in syringe 7A is pushed in downward. The inside of hollow shaped section 7C of injection needle 7BB is pressurized by an air pressure generated at this time. Accordingly, solid gel 4H in hollow shaped section 7C is pressed downward and extruded to the outside of hollow shaped section 7C. Note that, at this time, an upper part of container 9A is closed by gel plate 6P and a space in container 9A is formed as a closed chamber. The closed chamber is preferably filled with PBS or a culture solution.

Fig. 66 is a schematic sectional view illustrating a modification of the eighth step illustrated in Fig. 65. Referring to Fig. 66, since a form illustrated here is basically the same as the form illustrated in Fig. 65, the same components as the components illustrated in Fig. 65 are denoted by the same reference numerals and signs and explanation of the components is not repeated as long as functions and the like of the components are the same. However, in Fig. 66, substrate 9B installed in container 9A is used as the recovering section. Solid gel 4H discharged from the inside of hollow shaped section 7C is recovered on the upper surface of substrate 9B. In this regard, Fig. 66 is different in a configuration from Fig. 65 in which container 9A itself is the recovering section. Such a form may be adopted as long as the upper surface of substrate 9B is capable of receiving solid gel 4H discharged from the inside of hollow shaped section 7C.

### (Details about the solid gel)

Solid gel 4H extruded as explained above has a pillar shape. For example, if the thickness of spacer 3S is 100 µm and the diameter of the cross section of hollow shaped section 7C (see Fig. 3) of injection needle 7BB is approximately 600 µm, solid gel 4H is formed in a columnar shape, the diameter of a circular bottom surface of which is approximately 600 µm and the height of which is 100 µm. However, not only this, but solid gel 4H to be formed is formed in a prism shape if the cross section of hollow shaped section 7C of injection needle 7BB has a polygonal shape. In any case, it is preferable that cut solid gel 4H has a columnar or a polygonal prism (pillar) shape. That is, if the cross section of formed solid gel 4H is cut, the cross section has a uniform shape with balance kept as a whole, for example, symmetrical with respect to a center line. When the diameter of the bottom surface is larger than the height as in the example explained above, solid gel 4H has a tablet shape. By using spacer 3S and injector 7 as explained above, it is possible to cut a sample having any size and shape. It is possible to form solid gel 4H, which is uniform in the dimensions such as width and thickness (height).

### (Example of factor concentration gradient formation in the container)

### (First example)

Fig. 67 is a schematic sectional view illustrating a step equivalent to the eighth step illustrated in Fig. 65 in a first example of supply of the solid gel into the container. Referring to Fig. 67, a culture solution 13 serving as a medium in which a cell is cultured is stored in container 9A. In relation to the explanation referring to Fig. 65, the piston of injector 7 is pushed in downward in a state in which injection needle 7BB is immersed in culture solution 13. Accordingly, solid gel 4H is extruded and supplied into container 9A (into culture solution 13).

In Fig. 67, factors 4G contained in solid gel 4H are indicated by circles one by one from the viewpoint of easily imaging factors 4G. However, actually, factors 4G are not limited to such a shape. For example, factors 4G may be integrated as a whole. It is premised that factors 4G are uniformly distributed in solid gel 4H irrespective of the shape of solid gel 4H.

Fig. 68 is a schematic sectional view illustrating a culture implement formed by a step following Fig. 67 in the first example of the supply of the solid gel into the container. Referring to Fig. 68, if extruded solid gel 4H is left to stand in culture solution 13, a state in which a fluorescent dye is sustainedly released from gel 4D can be observed by a fluorescence microscope. This is because a dye having fluorescence is mixed in gel material 4A. A concentration gradient 15 of factors 4G emitted from solid gel 4H can be visually recognized in the periphery of solid gel 4H. Concentration gradient 15 is formed radially centering on solid gel 4H. Factors 4G have higher concentration in a region closer to solid gel 4H and have lower concentration further away from solid gel 4H. If a change in fluorescence intensity at this time is measured, a sustained-release ability of solid gel 4H as a sustained-release material can be evaluated. In a system illustrated in Fig. 67 and Fig. 68, although not illustrated in Fig. 67 and Fig. 68, if culture of a cell is completed, a culture implement 17 is formed. Solid gel 4H remaining in culture implement 17 illustrated in Fig. 68 is placed on the bottom surface of container 9A. Note that, actually, it is also assumed that solid gel does not remain in culture implement 17. However, in the present embodiment, it is premised that solid gel 4H remains in culture implement 17 serving as a product after the culture completion.

### (Second example)

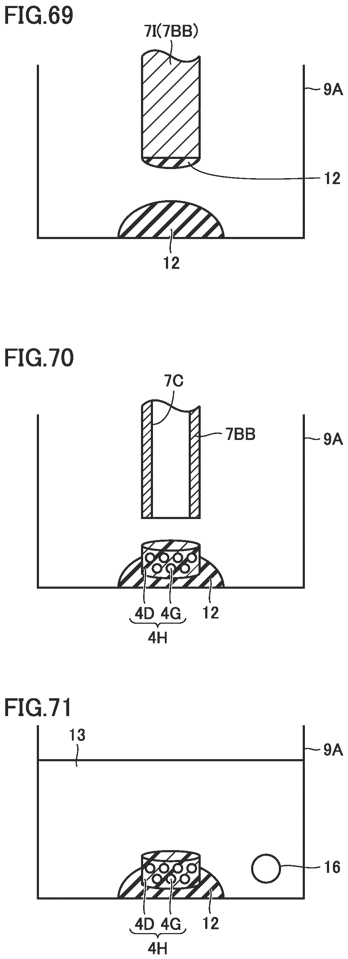

Fig. 69 is a schematic sectional view illustrating a step equivalent to a pre-stage of the eighth step illustrated in Fig. 65 in a second example of the supply of the solid gel into the container. Referring to Fig. 69, in the second example, first, a high viscosity solution 12 is supplied into container 9A (for example, the bottom surface). As high viscosity solution 12, it is preferable that, for example, cellulose, cellulose nanofiber, methylcellulose, carboxymethylcellulose, hydroxybutylcellulose, sodium alginate, sodium hyaluronate, or polyethylene glycol is used. High viscosity solution 12 may be supplied by injection needle 7BB including hollow shaped section 7C explained above but may be supplied by a solid needle 7I different from injection needle 7BB.

Fig. 70 is a schematic sectional view illustrating a step equivalent to the eighth step illustrated in Fig. 65 in the second example of the supply of the solid gel into the container. Referring to Fig. 70, as in Fig. 65 and Fig. 67, solid gel 4H cut by injection needle 7BB is supplied into container 9A and fixed. At this time, solid gel 4H is superimposed on high viscosity solution 12, whereby solid gel 4H is fixed to container 9A.

Fig. 71 is a schematic sectional view illustrating a step following Fig. 70 in the second example of the supply of the solid gel into the container. Referring to Fig. 71, after solid gel 4H is fixed, culture solution 13 serving as a medium for culture is supplied into container 9A. For example, culture solution 13 is supplied to fill most of the inside of container 9A. Culture solution 13 immerses solid gel 4H and covers the surface of solid gel 4H. A mass of a cell 16 to be cultured is supplied into container 9A together with culture solution 13.

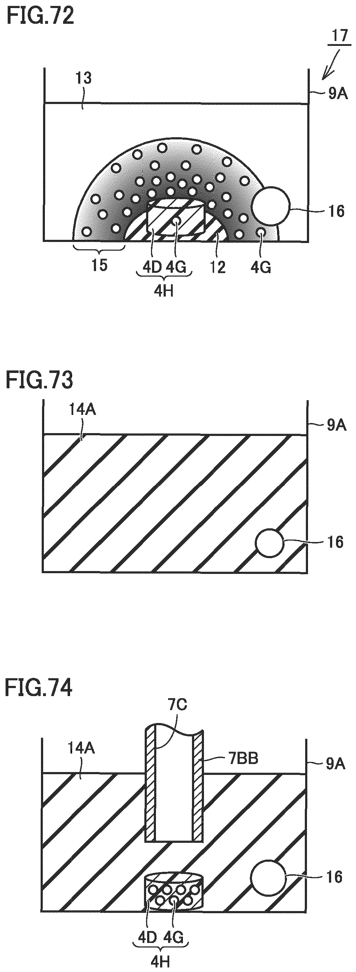

Fig. 72 is a schematic sectional view illustrating a culture implement formed by a step following Fig. 71 in the second example of the supply of the solid gel into the container. Referring to Fig. 72, a part of factors 4G in solid gel 4H is emitted to the outside of solid gel 4H and concentration gradient 15 of factors 4G is formed in culture solution 13.

When concentration gradient 15 is formed as illustrated in Fig. 72, at the same time, cell 16 is cultured and, for example, grown and proliferated. In Fig. 72, cell 16 cultured by the growth, the proliferation, and the like is indicated by cell 16 larger than cell 16 illustrated in Fig. 71. That is, in the second example, before culture of a cell is started, factors 4G are supplied into container 9A, and concentration gradient 15 of factors 4G is formed in culture solution 13 simultaneously with the culture of cell 16. The culture of cell 16 is completed, whereby culture implement 17 is obtained.

### (Third example)

Fig. 73 is a schematic sectional view illustrating a step equivalent to the pre-stage of the eighth step illustrated in Fig. 65 in a third example of the supply of the solid gel into the container. In the third example, a situation in which the culture of cell 16 has already been started in container 9A at an initial stage illustrated in Fig. 73 is assumed. This is because the culture of cell 16 is possible even if a factor is absent. Referring to Fig. 73, here, a gel precursor 14A serving as a medium for culture is filled in container 9A. The material of gel precursor 14A is a substance including at least one or more kinds selected out of a group consisting of collagen, atelocollagen, gelatin, fibrin, hyaluronic acid, Matrigel, agarose, alginic acid, chitin, chitosan, polyethylene glycol, polyvinyl alcohol, polyacrylamide, and dielectrics of the above substances.

Fig. 74 is a schematic sectional view illustrating a step equivalent to the eighth step illustrated in Fig. 65 in the third example of the supply of the solid gel into the container. Referring to Fig. 74, as in Fig. 65 and Fig. 67, solid gel 4H cut by injection needle 7BB is supplied into container 9A. The piston of injector 7 is pushed in downward in a state in which injection needle 7BB is immersed in gel precursor 14A. Accordingly, solid gel 4H is extruded and supplied into container 9A (into gel precursor 14A). Solid gel 4H is placed on the bottom surface in container 9A.

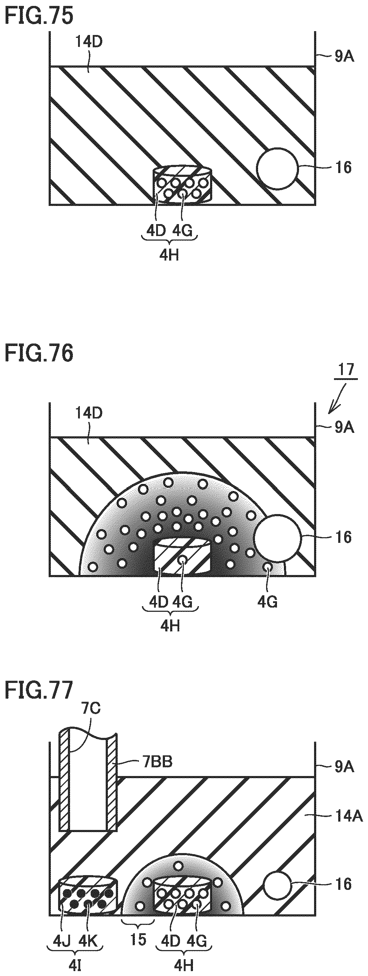

Fig. 75 is a schematic sectional view illustrating a step following Fig. 74 in the third example of the supply of the solid gel into the container. Referring to Fig. 75, after solid gel 4H is supplied, gel precursor 14A in container 9A is cooled or heated to thereby be gelatinized (solidified) and changes to gel 14D. That is, if gel precursor 14A is a material solidified by cooling like agarose and gelatine, gel precursor 14A is gelatinized by being cooled to, for example, 4°C. On the other hand, if gel precursor 14A is a material solidified by heating like collagen, gel precursor 14A is gelatinized by being heated to, for example, 37°C. Solidified gel 14D surrounds the periphery of solid gel 4H and embeds solid gel 4H. Accordingly, solid gel 4H is fixed on the bottom surface of container 9A.

Fig. 76 is a schematic sectional view illustrating a culture implement formed by a step following Fig. 75 in the third example of the supply of the solid gel into the container. Referring to Fig. 76, a part of factors 4G in solid gel 4H is emitted to the outside of solid gel 4H. Concentration gradient 15 of factors 4G is formed in the periphery of solid gel 4H in gel 14D. As illustrated in Fig. 75 to Fig. 76, even in gel 14D obtained by solidifying gel precursor 14A, the sustained-release of factors 4G, the formation of concentration gradient 15, and the culture of cell 16 are possible.

When the culture of cell 16 is completed in container 9A, culture implement 17 is formed. Solid gel 4H remaining in culture implement 17 illustrated in Fig. 76 has a pillar shape and is covered with gel 14D.

In the present embodiment, as in the second example, solid gel 4H may be supplied before cell 16 is started to be cultured. Alternatively, although not illustrated, in the second example, cell 16 and solid gel 4H may be simultaneously supplied into container 9A. As in the third example, after the culture of cell 16 is started, solid gel 4H may be supplied at appropriate timing during the culture. That is, a step of supplying solid gel 4H into container 9A is performed at any timing at or after a point in time before the cell is started to be cultured in container 9A. Any timing is, specifically, any one of before the cell culture start, at the time of the cell culture start, and after the cell culture start. Note that, when solid gel 4H is supplied before the culture start, it is preferable that solid gel 4H is supplied in or after a time one minute or more and twelve hours or less than cell 16 is supplied.

In the second example, the culture of cell 16 in container 9A and the formation of concentration gradient 15 are substantially simultaneously started. On the other hand, in the third example, concentration gradient 15 of factors 4G is formed from solid gel 4H while the culture of cell 16 is in progress in container 9A. It is considered from the second example and the third example that concentration gradient 15 of factors 4G can be formed in a medium from solid gel 4H at any timing halfway in cell 16 being cultured in container 9A. In the next fourth example and fifth example, an example in which factors are put in and a concentration gradient is formed during culture of a cell as in the third example is explained more in detail and specifically.

### (Fourth example)

Fig. 77 is a schematic sectional view illustrating a step equivalent to Fig. 74 in a fourth example of the supply of the solid gel into the container. Referring to Fig. 77, in the fourth example, factors 4G are sustainedly released from solid gel 4H of container 9A and concentration gradient 15 is formed in gel precursor 14A and, while culture of cell 16 is performed, solid gel 4I different from solid gel 4H is further supplied into container 9A (gel precursor 14A). Like solid gel 4H, solid gel 4I includes gel 4J and factors 4K contained in gel 4J. Like solid gel 4H, solid gel 4I is discharged from injection needle 7BB of sample collecting apparatus 100 to thereby be formed to have a pillar shape. The material of gel 4J is selected out of the same group as the group from which the material of gel 4D is selected. The material of factors 4K is selected out of the same group as the group from which the material of factors 4G is selected. However, at least a type of factors 4K and a type of factors 4G are different from each other. Solid gel 4I is disposed at an interval from solid gel 4H in container 9A.

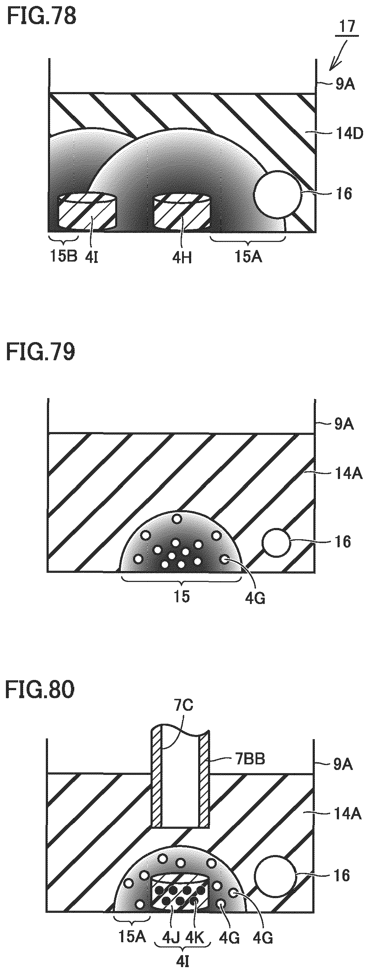

Fig. 78 is a schematic sectional view illustrating a culture implement formed by a step equivalent to Fig. 76 in the fourth example of the supply of the solid gel into the container. Referring to Fig. 78, in the fourth example, the same treatment as the treatment illustrated in Fig. 75 and Fig. 76 in the third example is performed. That is, a concentration gradient 15B of factors 4K is formed in the periphery of solid gel 4I by sustained-release of factors 4K contained in solid gel 4I. A concentration gradient 15A of factors 4G is formed in the periphery of solid gel 4H by sustained-release of factors 4G contained in solid gel 4H. If the culture of cell 16 is completed under such an environment, culture implement 17 is formed. Culture implement 17 illustrated in Fig. 78 includes factors 4G and factors 4K. Culture implement 17 includes solid gel 4H and solid gel 4I, which are arranged in the lateral direction to be covered with gel 14D. Gel precursor 14A is gelatinized to be gel 14D. Solid gels 4H and 4I are fixed in container 9A by gel 14D surrounding solid gels 4H and 4I.

### (Fifth example)

Fig. 79 is a schematic sectional view illustrating a step equivalent to a pre-stage of Fig. 77 in a fifth example of the supply of the solid gel into the container.
Referring to Fig. 79, in the fifth example, as in the fourth example, two factors and two solid gels are used. However, in Fig. 79, factors 4G are sustainedly released from solid gel 4H of container 9A, concentration gradient 15 is formed in gel precursor 14A, and solid gel 4H is removed from the inside of container 9A while the culture of cell 16 is performed.

Fig. 80 is a schematic sectional view illustrating a step equivalent to Fig. 77 in the fifth example of the supply of the solid gel into the container. Referring to Fig. 80, solid gel 4I different from solid gel 4H is supplied to the same place as a place where solid gel 4H was placed before a point in time of Fig. 79 in container 9A (gel precursor 14A). Solid gel 4I in the fifth example is the same as solid gel 4I in the fourth example.

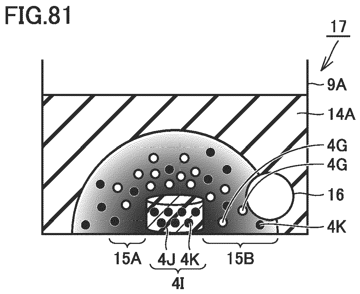

Fig. 81 is a schematic sectional view illustrating a culture implement formed by a step equivalent to Fig. 78 in the fifth example of the supply of the solid gel into the container. Referring to Fig. 81, thereafter, factors 4K are sustainedly released from solid gel 4I as in the other examples explained above. In culture implement 17 after culture completion in Fig. 81, concentration gradient 15A by factors 4G and concentration gradient 15B by factors 4K are formed in a concentric circle shape (to at least partially overlap). For example, as illustrated in Fig. 81, concentration gradient 15B may be larger in size than concentration gradient 15A. Alternatively, concentration gradient 15A and concentration gradient 15B may be substantially the same in size and concentration.

### (Action effects)

In the method for manufacturing the culture implement according to the present embodiment, gels 4D and 4J containing factors 4G and 4K to be supplied are formed on film 2 in a sheet state and gel sheet 4 is provided on film 2. Gel sheet 4 on film 2 is punctured by the needle (injection needle 7BB) including hollow shaped section 7C to cut a part of gel sheet 4. Solid gels 4H and 4I, which are cut portions of gel sheet 4, are stored in hollow shaped section 7C. Solid gels 4H and 4I in hollow shaped section 7C are discharged from the inside of hollow shaped section 7C, whereby solid gels 4H and 4I are supplied, as a sustained-release material including factors 4G and 4K, into container 9A in which cell 16 is cultured.

If the method explained above is used, it is possible to control the size of solid gel cut by injection needle 7BB and timing when the solid gel is supplied into container 9A. Accordingly, it is possible to control timing when concentration gradient 15 is formed and a disposition amount of factors in concentration gradient 15 (the positions and the density of the factors in culture implement 17). Since concentration gradient 15 can be easily controlled, it is possible to provide highly accurate culture implement 17 including a biologically similar structure more similar to an organism.

In the method for manufacturing the culture implement explained above, when solid gels 4H and 4I are supplied, a medium for cell 16 to be cultured and a sustained-release material to be emitted (culture solution 13 serving as liquid or gel precursor 14A) is supplied into container 9A. The sustained-release material forms a concentration gradient of factors 4G and 4K in the medium at any timing halfway in cell 16 being cultured in container 9A.

In order to make timing when concentration gradient 15 is formed optional, for example, it is possible to optionally select whether solid gel 4H is disposed before the start of the cell culture as in the second example or solid gel 4H is disposed during the cell culture as in the third example. Accordingly, it is possible to provide highly accurate culture implement 17 including a biologically similar structure more similar to an organism. This is because concentration gradient 15 can control a behavior of cell 16 that migrates or infiltrates in the medium. Further, this is because differentiation and structure formation in an organoid and a three-dimensional tissue can be controlled according to concentration gradient 15.

In the method for manufacturing the culture implement, the factors includes first factors (factors 4G) and second factors (factors 4K). The step of supplying solid gels 4H and 4I includes a step of supplying the first factors (factors 4G) into container 9A at first time t1 and a step of supplying the second factors (factors 4K) into container 9A at second time t2 later than the first time t1.

Second factors 4K can be supplied, for example, at the second time in the middle when cell 16 is cultured while first factors 4G is sustainedly released in the medium in container 9A. By optionally changing second time t2, it is possible to control the factors and the like in finally formed concentration gradients 15A and 15B. For this reason, it is possible to provide highly accurate culture implement 17 including a biologically similar structure more similar to an organism. As an example, it is possible to control differentiation of cell 16 by adding the second factors 4K after one day or more and seven days or less elapse from the start of culture of cell 16 by the first factors 4G.

In the method for manufacturing the culture implement, solid gels 4H and 4I cut in the step of supplying solid gels 4H and 4I may be fixed to container 9A.

Accordingly, it is possible to highly accurately control the positions of the factors (if the factors are uniformly contained). For this reason, it is possible to form highly accurate concentration gradient 15 and provide highly accurate culture implement 17. Note that, here, the solid gel being fixed to container 9A means that the solid gel may be fixed by high viscosity solution 12 as illustrated in Fig. 71 or may be fixed by gel 14D as illustrated in Fig. 75. The solid gel being fixed container 9A is not limited to a case in which the solid gel is in direct contact with container 9A and includes a case in which the solid gel is recovered and fixed on substrate 9B installed in container 9A as illustrated in Fig. 66.

In the method for manufacturing the culture implement, solid gels 4H and 4I cut in the step of storing solid gels 4H and 4I may have a pillar shape.

Since solid gels 4H and 4I have a symmetrical and uniform shape such as the pillar shape, an amount, sustained-release speed, and a sustained-release amount of factors 4G and 4K contained in solid gels 4H and 4I become uniform. This is because it is premised that factors 4G are uniformly distributed in solid gel 4H. For this reason, it is possible to control the parameters of factors 4G and 4K not to be an unintended amount and speed. Therefore, it is possible to control the factors and the like in finally formed concentration gradients 15A and 15B. For this reason, it is possible to provide highly accurate culture implement 17 including a biologically similar structure more similar to an organism.

In the method for manufacturing the culture implement, in the step of supplying solid gels 4H and 4I, a gel sheet portion (solid gels 4H and 4I) cut by injection needle 7BB and a portion of film 2 disposed right under the gel sheet portion may be penetrated. After the penetrating step, the solid gel may be recovered in the recovering section (container 9A: substrate 9B is also possible). Film 2 is used in gel plate 6P. For this reason, injection needle 7BB can cut and penetrate gel 4D and easily break film 2 that is in contact on the lower side of gel 4D and penetrate film 2. Accordingly, it is possible to perform sample collection with high stability, reproducibility, and position accuracy.

In culture implement 17 according to the present embodiment, cell 16 is cultured. Culture implement 17 includes container 9A, solid gels 4H and 4I, and the medium (culture solution 13 serving as liquid or gel precursor 14A) in which cell 16 is cultured. Solid gels 4H and 4I are fixed in container 9A and factors 4G and 4K are contained in solid gels 4H and 4I. The medium fills at least a part in container 9A and cell 16 is cultured. Solid gels 4H and 4I are disposed to be covered with the medium. Solid gels 4H and 4I have a pillar shape.

Since solid gels 4H and 4I have a symmetrical and uniform shape such as a pillar shape, an amount, sustained-release speed, and a sustained-release amount of factors 4G and 4K contained in solid gels 4H and 4I become uniform. For this reason, the parameters of factors 4G and 4K can be controlled not to be an unintended amount and speed. Therefore, it is possible to easily control the factors and the like of finally formed concentration gradients 15A and 15B. For this reason, it is possible to provide highly accurate culture implement 17 including a biologically similar structure more similar to an organism.

In culture implement 17, concentration gradients of factors 4G and 4K are formed in the peripheries of solid gels 4H and 4I.

Highly accurate culture implement 17 including a biologically similar structure more similar to an organism can be provided by concentration gradient 15. This is because concentration gradient 15 can control a behavior of cell 16 that migrates or infiltrates in the medium. Further, this is because differentiation and structure formation in an organoid and a three-dimensional tissue can be controlled according to concentration gradient 15.

In culture implement 17, the factors include first factors (factors 4G) and second factors (factors 4K). The solid gel includes first solid gel (solid gel 4H) containing the first factors (factors 4G) and second solid gel (solid gel 4I) containing the second factors (factors 4K). The first solid gel 4H and the second solid gel 4I are arranged to be covered with the medium (see Fig. 78).

Culture implement 17 having such a configuration can be formed because the second factors can be supplied, for example, at second time in the middle when cell 16 is cultured while the first factors is sustainedly released in the medium in container 9A. By optionally changing the second time, it is possible to control the factors and the like in finally formed concentration gradients 15A and 15B. For this reason, it is possible to provide highly accurate culture implement 17 including a biologically similar structure more similar to an organism.

In culture implement 17, solid gels 4H and 4I may be fixed to container 9A by high viscosity solution 12.

If the sustained-release material is only left to stand in a solution, it is likely that the position of the sustained-release material moves because of swing and vibration at the time of transportation, making it difficult to maintain a state in which concentration gradient 15 is controlled. For this reason, if high viscosity solution 12 is used, it is possible to suppress unintended movement of the positions of solid gels 4H and 4I in a medium due to vibration and swing. It is possible to suppress a deficiency such as positional deviation of concentration gradient 15 involved in the unintended movement.

In culture implement 17 explained above, solid gels 4H and 4I may be fixed to container 9A by gel 14D surrounding the peripheries of solid gels 4H and 4I.

When gel 14D is used, as in the case in which high viscosity solution 12 is used, there is an effect of suppressing unintended movement of the positions of solid gels 4H and 4I. In addition, if gel 14D is used, it is possible to suppress a deficiency in which a medium itself in the peripheries of solid gels 4H and 4I causes vibration and swing, making it difficult to maintain and control the position and the concentration of concentration gradient 15 formed in the medium.

In culture implement 17, solid gels 4H and 4I may be placed on the bottom surface in container 9A.

Accordingly, it is possible to more stably fix solid gels 4H and 4I to container 9A compared with, for example, when solid gels 4H and 4I are fixed to a plane extending in the Z direction of container 9A.

The characteristics of the examples and the like described in the embodiments explained above may be applied to be combined as appropriate in a range without technical contradiction.

The embodiments disclosed herein should be considered as illustrative and not limiting in all aspects. The scope of the present invention is indicated not by the above explanation but by the claims. It is intended that all changes in meanings and ranges equivalent to the claims are included in the scope of the present invention.

### REFERENCE SIGNS LIST

1, 5, 5A Slide glass; 2 Film; 3 Frame; 3A, 3C Through-hole; 3B Another frame; 3S Spacer; 4 Gel sheet; 4A Gel material; 4B Sample; 4C Fluid sample; 4D, 4J, 14D Gel; 4E Gel sheet portion; 4G, 4K Factor; 4H, 4I Solid gel; 6, 6A, 6B Gel sheet chip; 6P Gel plate; 7A Syringe; 7B Pin; 7BB Injection needle; 7C Hollow shaped section; 7CP Syringe cap; 7D Upper syringe fixing section; 7E Lower syringe fixing section; 7F Holder outer shell; 7G Tube; 7H Collecting needle holder; 71 Solid needle; 8 Plate; 9A Container; 10, 10A Gel dissolving agent; 11 Sample collecting set; 12 High viscosity solution; 13 Culture solution; 14A Gel precursor; 15 Concentration gradient; 16 Cell; 17 Culture implement; 20 Culture solution; 21 Sheet fixing member; 40 First driving unit; 41 Servomotor; 43 Cam; 44 Bearing; 45 Cam coupling plate; 46 Movable unit; 100 Sample collecting apparatus; 101 XY stage for sample; 101A Stage penetrating section; 102 XY stage for container; 104 Collecting mechanism; 106 Observation optical system.

## Claims

1. A gel sheet chip to which a sample to be collected is fixed, the gel sheet chip comprising:
a film;
a frame placed on the film, a through-hole being formed in the frame;
gel disposed on the film in the through-hole; and
the sample to be collected supported by the gel.

2. The gel sheet chip according to claim 1, wherein thickness of the gel is equal to or less than thickness of the frame.

3. The gel sheet chip according to claim 1 or 2, wherein the sample is disposed on an upper surface of the gel to adhere to the gel.

4. The gel sheet chip according to claim 3, further comprising another frame placed on the frame to surround the sample on an upper surface of the gel.

5. The gel sheet chip according to claim 1 or 2, wherein the sample is disposed in the gel in the through-hole.

6. The gel sheet chip according to any one of claims 1 to 5, wherein the sample is a cell or a cell aggregate.

7. The gel sheet chip according to any one of claims 1 to 6, wherein the gel is made of a material having adhesion to a cell.

8. The gel sheet chip according to any one of claims 1 to 7, wherein the gel is gel obtained by gelatinizing a gel material having biocompatibility.

9. The gel sheet chip according to any one of claims 1 to 8, wherein the gel is made of a material having a characteristic of being decomposed and dissolved.

10. A method for manufacturing a gel sheet chip to which a sample to be collected is fixed, the method comprising:
installing, on a film, a frame in which a through-hole is formed;
supplying, into the through-hole on the film, a fluid sample including the sample to be collected;
uniformizing thickness of the fluid sample by sandwiching a structure including the film, the frame, and the fluid sample with flat plates installed on an upper side and a lower side of the structure; and
gelatinizing the fluid sample.

11. A sample collecting set for fixing a sample to be collected, the sample collecting set comprising:
a gel material capable of, by forming gel, supporting the sample to be collected;
a film on which the gel can be placed; and
a frame that can be placed on the film, a through-hole being formed in the frame.

12. A sample collecting method for collecting a fixed sample to be collected, the sample collecting method comprising:
forming, on a film, in a sheet state, gel that supports the sample to be collected and providing a gel sheet;
by the gel sheet on the film being punctured by a needle including a hollow shaped section, cutting a part of the gel sheet and storing a cut portion of the gel sheet in the hollow shaped section; and
collecting the portion of the gel sheet by discharging the portion of the gel sheet in the hollow shaped section from an inside of the hollow shaped section.

13. The sample collecting method according to claim 12, wherein the collecting includes:
penetrating the portion of the gel sheet cut by the needle and a portion of the film disposed right under the portion of the gel sheet; and
recovering the portion of the gel sheet in a recovering section after the penetrating.

14. The sample collecting method according to claim 13, wherein
the recovering section is a substrate or a container, and
the recovering section is disposed right under the needle.

15. The sample collecting method according to claim 13 or 14, wherein, in the recovering, only the portion of the gel sheet is recovered in the recovering section.

16. The sample collecting method according to any one of claims 12 to 15, wherein the portion of the gel sheet includes the gel and the sample supported in the gel.

17. The sample collecting method according to any one of claims 12 to 16, wherein
a plurality of the samples are included in the gel sheet, and
the sample collecting method further comprises, before the storing, dispersing the plurality of samples in the gel sheet to be disposed to be separated from one another.

18. The sample collecting method according to claim 17, wherein the isolated sample is included in the portion of the gel sheet stored in the hollow shaped section in the storing.

19. The sample collecting method according to any one of claims 12 to 16, wherein an aggregate of the sample formed by the plurality of samples being gathered is included in the portion of the gel sheet stored in the hollow shaped section in the storing.

20. The sample collecting method according to any one of claims 12 to 19, wherein the gel is gel obtained by gelatinizing a gel material having biocompatibility.

21. The sample collecting method according to any one of claims 12 to 20, further comprising:
dissolving the gel from the portion of the gel sheet; and
extracting the sample remaining after the dissolving.

22. A gel plate to which a sample to be collected is fixed, the gel plate comprising:
a film; and
a gel sheet formed on the film in a sheet state and including gel that supports the sample to be collected.

23. A sample collecting set for fixing a sample to be collected, the sample collecting set comprising:
a gel material capable of, by forming gel, supporting the sample to be collected; and
a film on which the gel can be placed.

24. A method for manufacturing a culture implement, the method comprising:
providing a gel sheet by forming, on a film, in a sheet state, gel containing a factor to be supplied;
by the gel sheet on the film being punctured by a needle including a hollow shaped section, cutting a part of the gel sheet and storing a cut portion of the gel sheet in the hollow shaped section; and
by discharging the solid gel in the hollow shaped section from an inside of the hollow shaped section, supplying the solid gel into, as a sustained-release material containing the factor, a container in which a cell is cultured.

25. The method for manufacturing the culture implement according to claim 24, wherein
in the supplying, a medium for the cell to be cultured and the sustained-release material being emitted is supplied into the container, and
the sustained-release material forms a concentration gradient of the factor in the medium at any timing halfway in the cell being cultured in the container.

26. The method for manufacturing the culture implement according to claim 25, wherein
the factor includes a first factor and a second factor, and
the supplying includes:
supplying the first factor into the container at first time; and
supplying the second factor into the container at second time later than the first time.

27. The method for manufacturing the culture implement according to any one of claims 24 to 26, wherein, in the supplying, the cut solid gel is fixed to the container.

28. The method for manufacturing the culture implement according to any one of claims 24 to 27, wherein, in the storing, the cut solid gel has a pillar shape.

29. The method for manufacturing the culture implement according to any one of claims 24 to 28, wherein the supplying includes:
penetrating the portion of the gel sheet cut by the needle and a portion of the film disposed right under the portion of the gel sheet; and
recovering the solid gel in the container after the penetrating.

30. The method for manufacturing the culture implement according to any one of claims 24 to 29, wherein the factor is any one selected out of a group consisting of protein, polysaccharide, and a low molecular weight compound.

31. The method for manufacturing the culture implement according to any one of claims 24 to 30, wherein the gel is gel obtained by gelatinizing a gel material having biocompatibility.

32. A culture implement in which a cell is cultured, the culture implement comprising:
a container;
solid gel fixed in the container, a factor being contained in the solid gel; and
a medium that fills at least a part in the container, the cell being cultured in the medium, wherein
the solid gel is disposed to be covered with the medium, and
the solid gel has a pillar shape.

33. The culture implement according to claim 32, wherein a concentration gradient of the factor is formed in a periphery of the solid gel.

34. The culture implement according to claim 33, wherein
the factor includes a first factor and a second factor,
the solid gel includes first solid gel containing the first factor and second solid gel containing the second factor, and
the first solid gel and the second solid gel are arranged to be covered with the medium.

35. The culture implement according to any one of claims 32 to 34, wherein the solid gel includes at least one type of a substance selected out of a group consisting of collagen, atelocollagen, gelatin, fibrin, hyaluronic acid, Matrigel, agarose, alginic acid, chitin, chitosan, polyethylene glycol, polyvinyl alcohol, acrylamide, and dielectrics of the above substances.

36. The culture implement according to any one of claims 32 to 35, wherein the factor is any one selected out of a group consisting of protein, polysaccharide, and a low molecular weight compound.

37. The culture implement according to any one of claims 32 to 36, wherein the solid gel is fixed to the container by a high viscosity solution.

38. The culture implement according to any one of claims 32 to 36, wherein the solid gel is fixed to the container by gel surrounding a periphery of the solid gel.

39. The culture implement according to any one of claims 32 to 38, wherein the solid gel is placed on a bottom surface in the container.
